# EUROPEAN PATENT SPECIFICATION

(11) **EP 0 294 910 B1**
(45) Date of publication and mention of the grant of the patent: **11.09.1996**
(21) Application number: 88201209.9
(22) Date of filing: 13.06.1988
(51) Int. Cl.: C12N 15/12, A61K 38/37, C07K 14/755, C12P 21/02, C12N 5/10, C12N 1/19, C12N 1/21

(54) **Novel proteins with factor VIII activity, process for their preparation using genetically engineered cells and pharmaceutical compositions containing them**
Proteine mit Faktor-VIII-Aktivität, Verfahren zu deren Herstellung unter Verwendung von genetisch modifizierten Zellen und diese enthaltende pharmazeutische Zusammensetzungen
Protéines à activité de facteur VIII, procédé pour leur production utilisant des cellules modifiées par génie génétique et compositions pharmaceutiques les contenant

(30) Priority: 12.06.1987 EP 87201121
(43) Date of publication of application: 14.12.1988
(60) Divisional application: 95110468.6
(73) Proprietor: IMMUNO AG, A-1221 Wien (AT)
(72) Inventor: van Ooyen, Albert Johannes Joseph, NL-2271 AR Voorburg (NL); Pannekoek, Hans c/o Centraal Laboratories van de, NL-1066 CX Amsterdam (NL); Verbeet, Martinus Philippus Centraal Laboratories, NL-1066 CX Amsterdam (NL); van Leen, Robert Willem, NL-6546 JW Nijmegen (NL)
(74) Representative: Davies, Jonathan Mark

(56) References cited:
- EP-A- 0 160 457
- EP-A- 0 232 112
- EP-A- 0 253 455
- EP-A- 0 265 778
- WO-A-86/06101
- WO-A-87/07144
- WO-A-88/00831
- BIOCHEMISTRY, vol. 25, no. 26, December 1986, pages 8343-8347, American Chemical Society, Washington, DC, US; D.L. EATON et al.: "Construction and characterization of an active factor VIII variant lacking the central one-third of the molecule"
- THE JOURNAL OF BIOLOGICAL CHEMISTRY, vol. 261, no. 27, 25th September 1986, pages 12574-12578, American Society of biological Chemists, Inc., US; R.L. BURKE et al.: "The functional domains of coagulation factor VIII:C"

## Description

### Technical Field

The invention relates to novel proteins having Factor VIII activity and methods for their preparation using genetically engineered cell-lines and microorganisms.

### Background

Hemophilia A is a sex-linked bleeding disorder characterized by a deficiency in Factor VIII, an essential element in the blood coagulation cascade. The disease occurs in about 0.01% of the male population. Hemophilia A can be treated by administering Factor VIII-containing blood plasma obtained from healthy donors. This treatment has several disadvantages however. The supply of Factor VIII is limited and very expensive; the concentration of Factor VIII in blood is only about 100 ng/ml and the yields using current plasma fractionation methods are low. Since the source of Factor VIII is pooled donor blood, the recipient runs a high risk of acquiring various infectious diseases, including those caused by hepatitis non-A, non-B, hepatitis B or AIDS viruses which may be present in the donor blood. In addition, recipients may develop antibodies against the exogenous Factor VIII, which can greatly reduce its effectiveness.

Factor VIII comprises three regions, an N-terminal region, the so-called "A1A2-domain"; a central region, the so-called "B domain"; and a C-terminal region comprising the A3, C1 and C2 domains. The A1A2-domain and the C-terminal region are believed essential for clotting activity. Factor VIII circulates in the blood combined with a protein, the von Willebrand factor (vWf), which is believed to protect the sensitive Factor VIII against early degradation.

Factor VIII is obtained in unsatisfactorily low yields when produced by known recombinant DNA processes. Moreover the proteins appear not to be present as an intact chain, hence products are difficult to isolate and to purify and consequently the costs are high. It is therefore desirable to develop an efficient way to produce large quantities of compounds having Factor VIII activity, the compounds preferably having decreased immunogenic activity.

### Relevant Literature

Molecular cloning of Factor VIII cDNA obtained from human mRNA and the subsequent production of proteins with Factor VIII activity in mammalian, yeast and bacterial cells has been reported. See WO 85/01961, EP 0160457, EP 0150735, EP 0253455. A method for producing proteins with Factor VIII activity using transformed microorganisms is disclosed in EP 0253455. European patent applications EP 0150735 and EP 0123945 and Brinkhous et al. (1985) disclose Factor VIII activity in proteolytic cleavage products of Factor VIII. A complex of two proteolytic cleavage products of Factor VIII, a 92 kDa and an 80 kDa polypeptide exhibits enhanced Factor VIII activity. (Fay et al., Biochem. Biophys. Acta (1986) 871:268-., 278; Eaton et al Biochemistry (1986) 25:505-512).

Eaton et al., Biochemistry (1986) 25:8343-8347 disclose that a polypeptide in which 766 amino acids (797-1562) have been deleted from the central region retains Factor VIII activity. Moreover, mammalian cells transformed with a vector comprising DNA encoding this deletion polypeptide had a higher production level than cells transformed with a vector comprising DNA encoding the full length polypeptide.

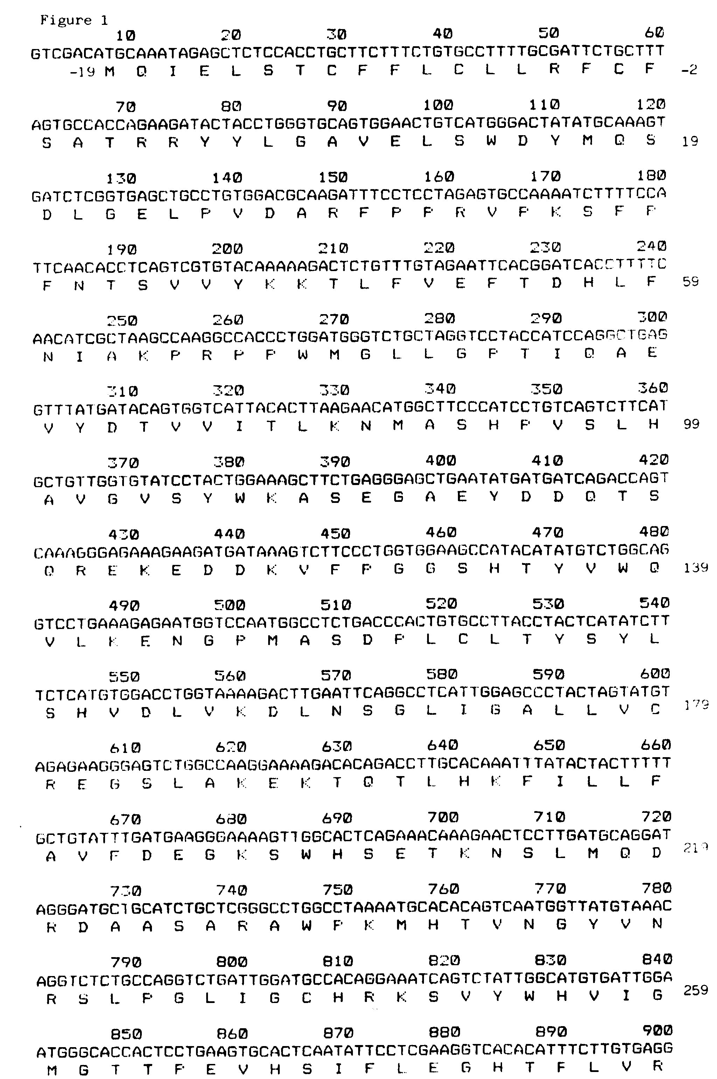

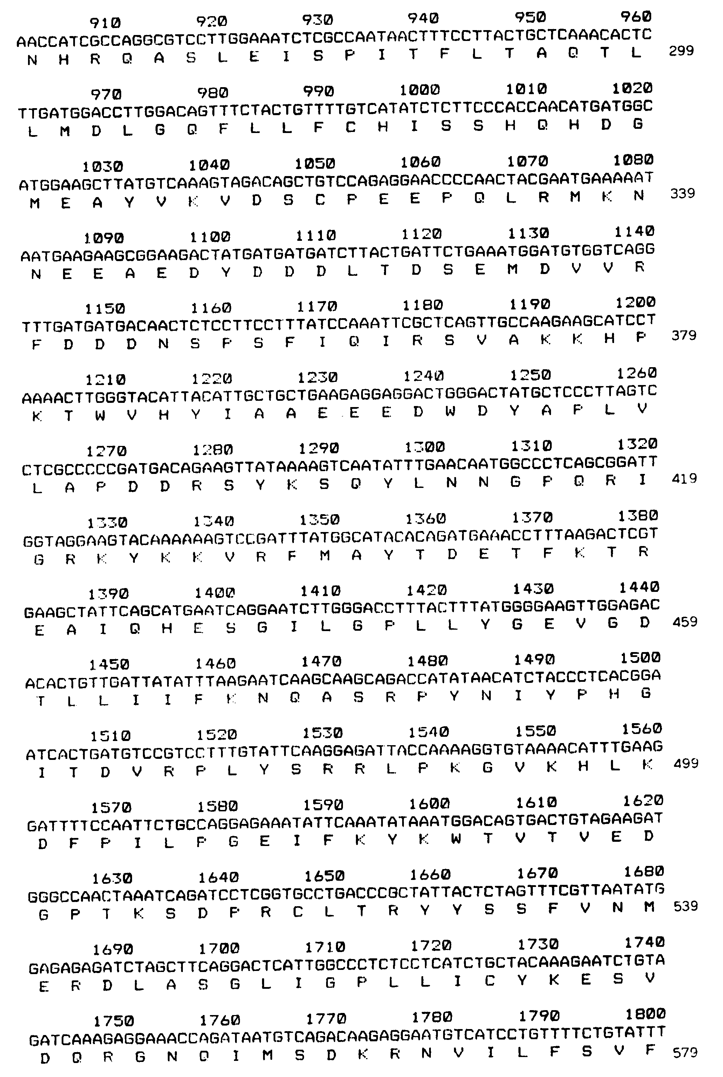

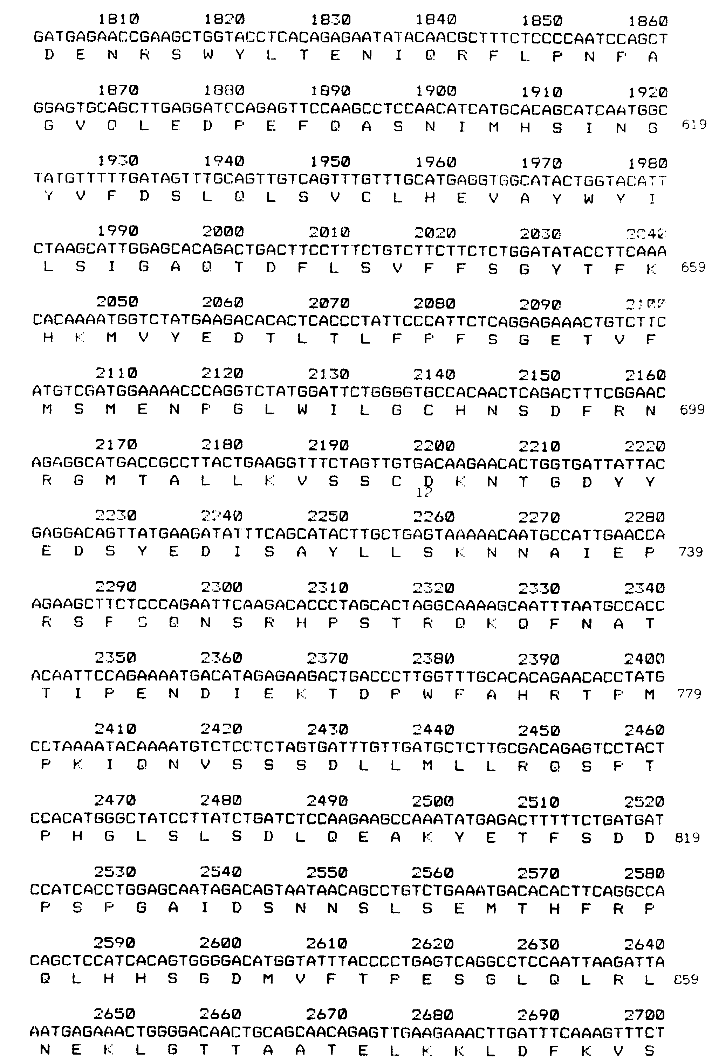

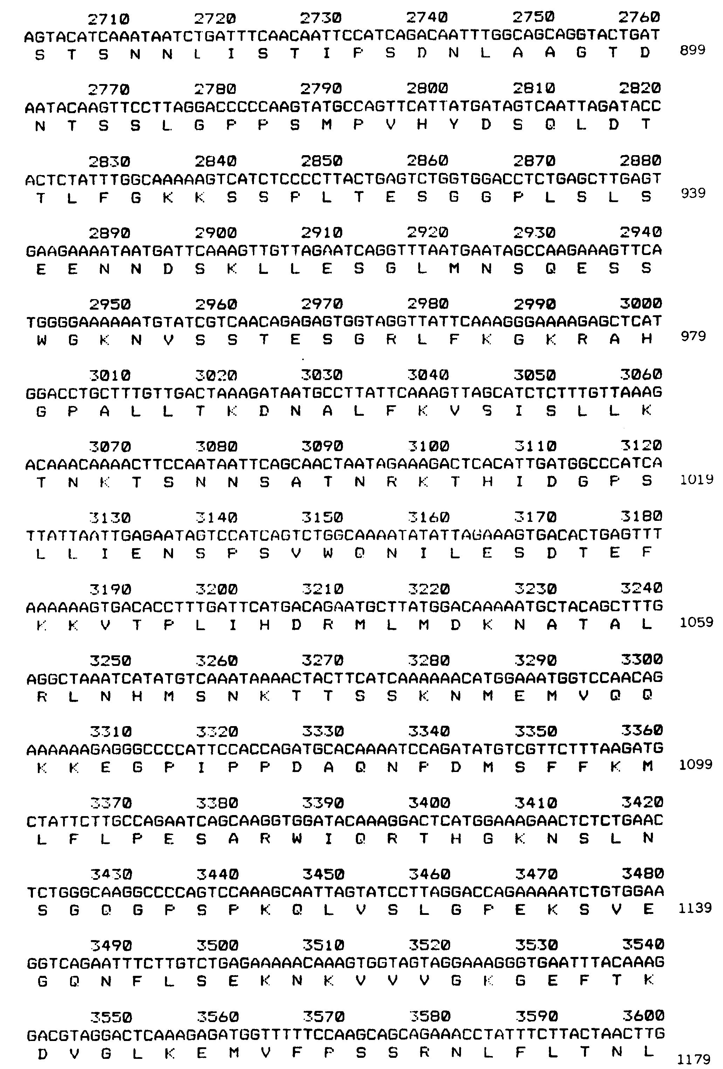

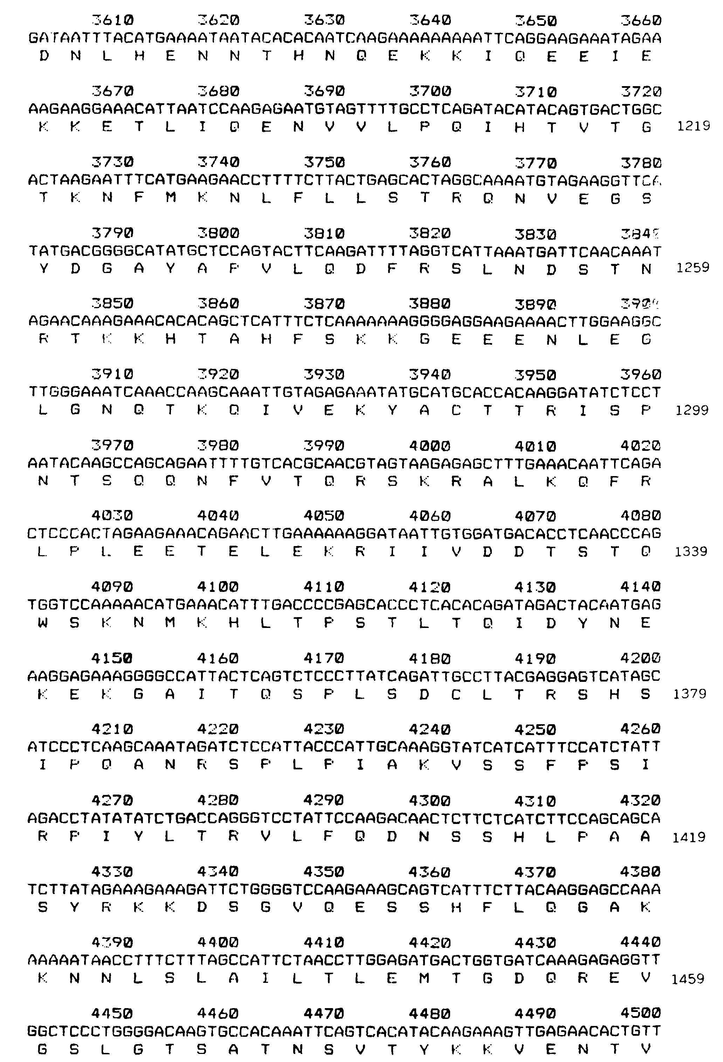

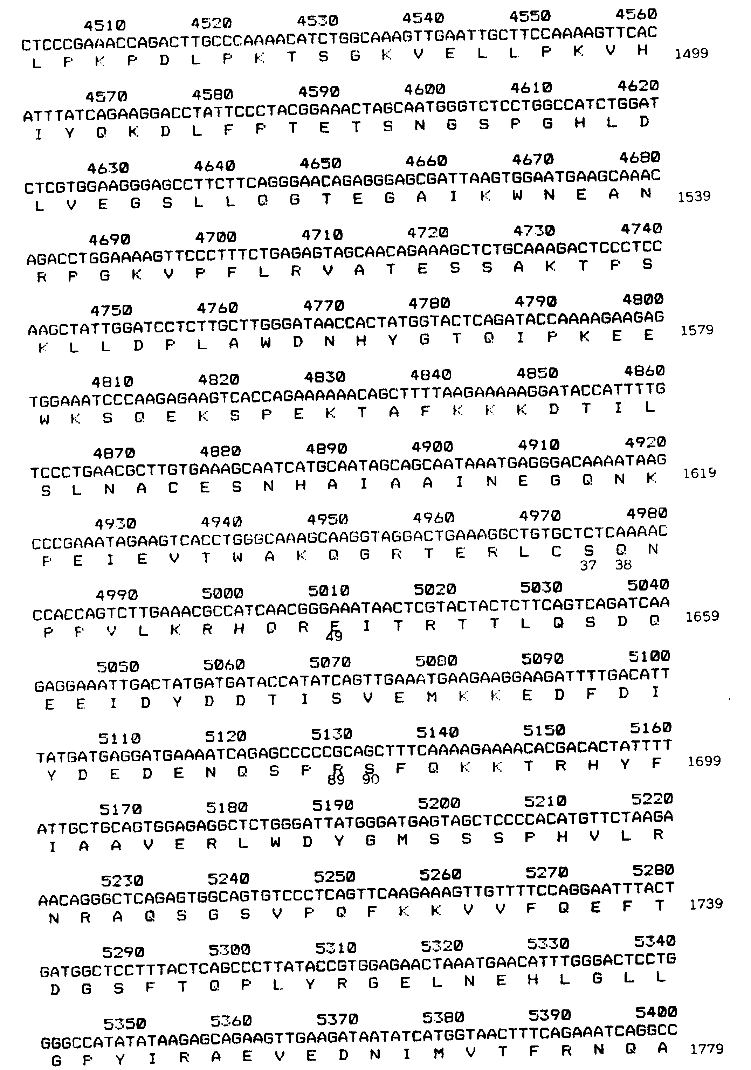

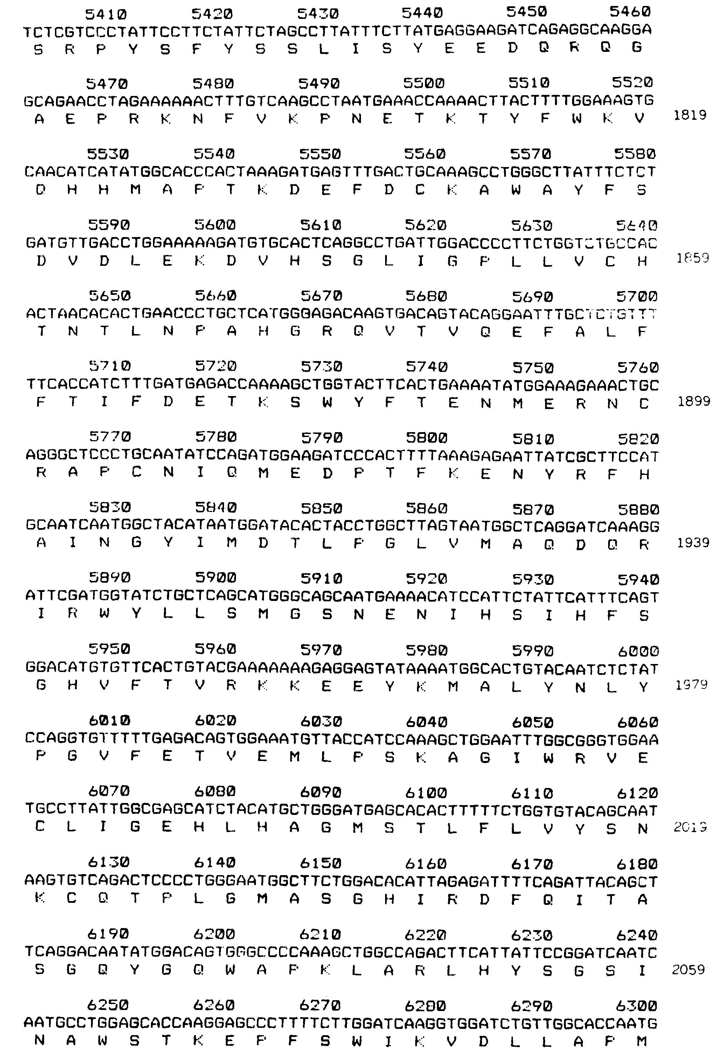

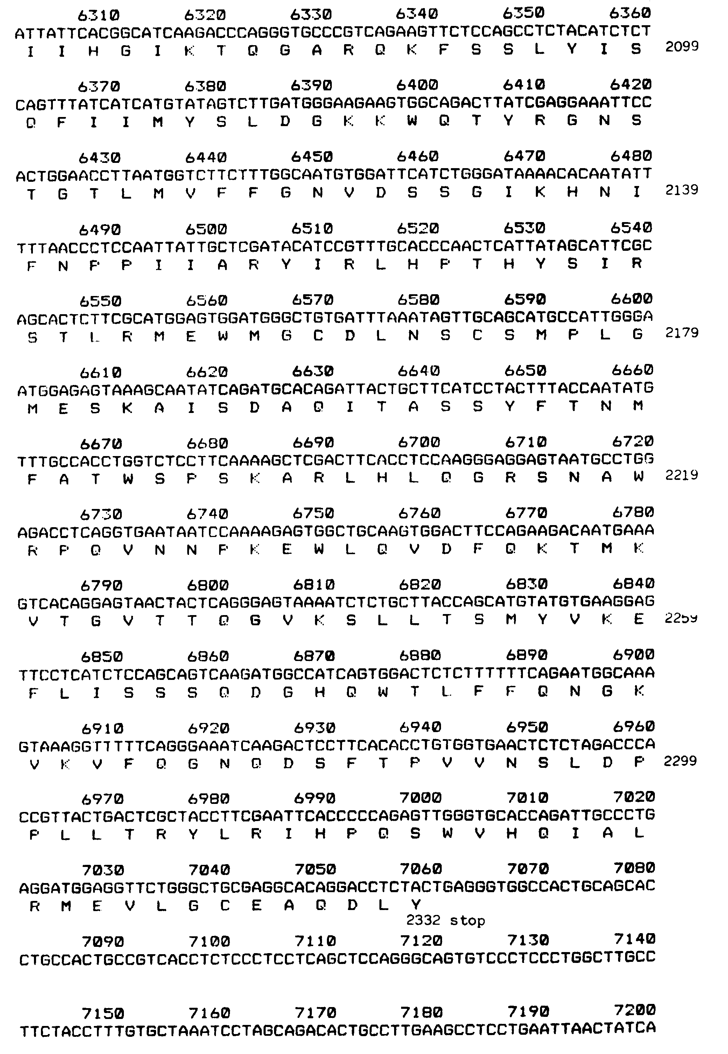

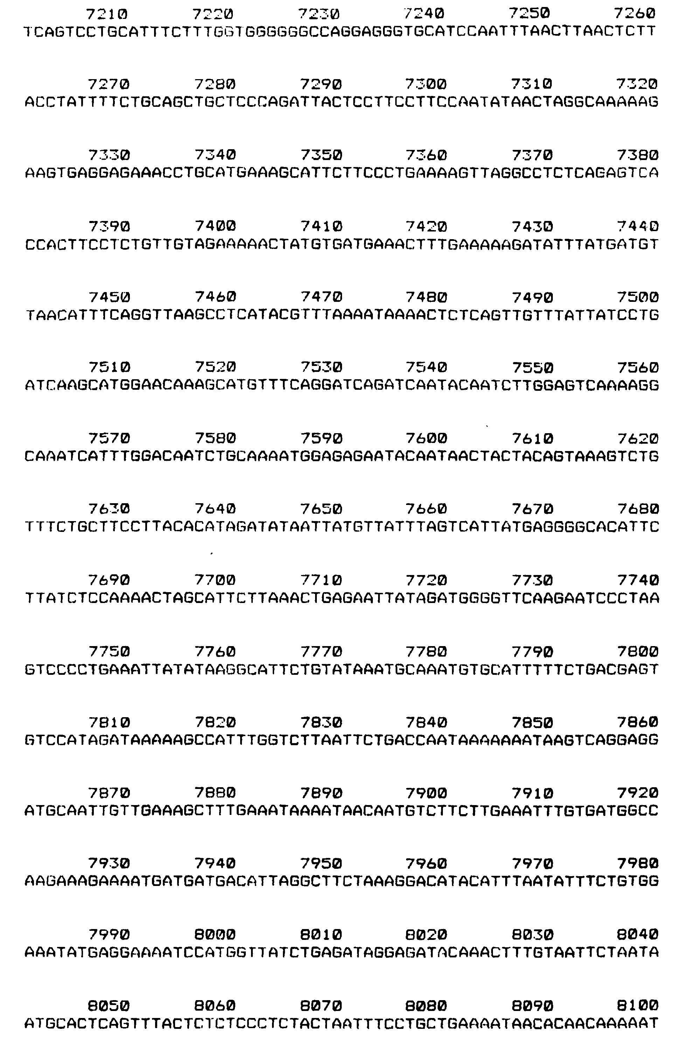

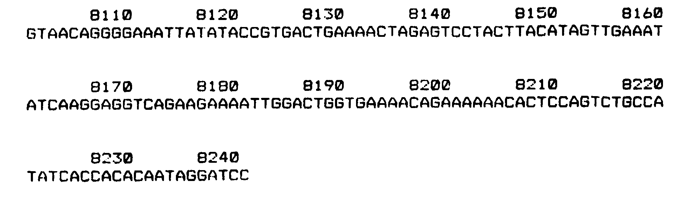

PCT application WO 86/06101 discloses that recombinant Factor VIII proteins with deletions of up to 880 amino acids in the central region exhibit Factor VIII activity. The largest deletion stretches from Thr-760 through Asn-1639 (numbering according to Figure 1). The host cells for preparation of the recombinant Factor VIII included mammalian cells, including Chinese hamster ovary cells.

### SUMMARY OF THE INVENTION

Novel compositions, together with expression vectors and methods for their preparation are provided, which comprise derivatives and fragments of Factor VIII. The compositions are prepared by transforming a host cell, preferably a mammalian cell, with an expression vector comprising a DNA sequence encoding a Factor VIII congener, growing the transformed host cell to express the exogenous DNA sequence, and recovering the resultant Factor VIII derivative or fragment from a cell lysate or from conditioned growth medium. The compositions may have enhanced Factor VIII activity and/or decreased immunogenicity. Uses of the compositions include treatment of Hemophilia A.

### BRIEF DESCRIPTION OF THE DRAWINGS

Figure 1 shows the Factor VIII cDNA insert of pCLB89 including the amino acid sequence Ala-1 through Tyr-2332 of Factor VIII and its signal sequence M(-19) through S(-1) with the corresponding sequence of nucleotides. F-973 is encoded by the codon TTC.

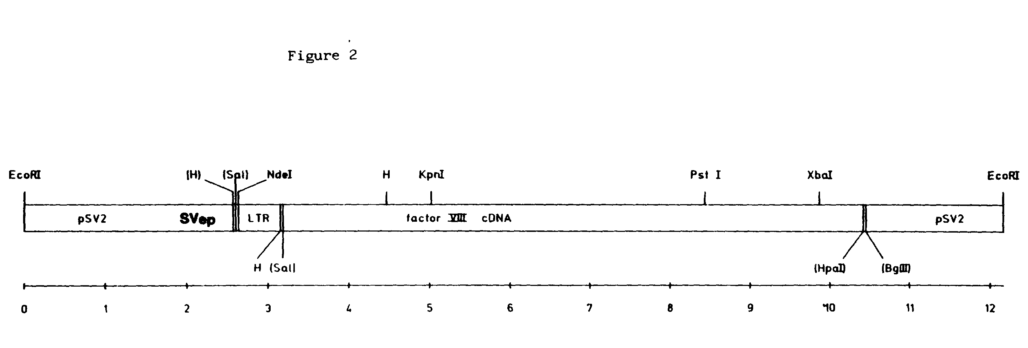

Figure 2 shows the structure of expression vector pCLB201 encoding the full-length Factor VIII protein. The circular plasmid pCLB201 is shown beginning with the EcoRI position within the 4217 bp HindIII-BglII fragment derived from plasmid pSV2 (Gorman, 1985 DNA Cloning (Ed. Glover) IRL Press, pp. 143-169; Mulligan and Berg, Proc. Natl. Acad. Sci. USA (1981) 78:2072). The early SV40-promoter region is indicated: SVep. The vector also contains a NdeI-HindIII fragment derived from plasmid pRSV-neo (Gorman, 1985, supra) bearing the Rous Sarcoma Virus-Long Terminal Repeat (LTR) inserted into a SalI site. The full-length Factor VIII is encoded by a SalI-HpaI fragment of 7440 bp and indicated: Factor VIII cDNA (H = HindIII, Sal = SalI). The restriction endonuclease cleavage sites lost during construction of pCLB201 are enclosed in parenthesis. The size of the plasmid is given in kilobase pairs (kb).

Figure 3 shows vectors for transient expression of deletion mutant Factor VIII proteins:
a. The landmarks of the pSV2-derived vector: two tandemly situated promoters: the SV40 early transcription promoter (SVep) and the Rous Sarcoma Virus-Long Terminal Repeat (RSV-LTR); the capping site (Capsite) and 5′ end of the messenger RNA (mRNA); the cDNA insert bearing the full-length Factor VIII coding region with the start codon (ATG), the open reading frame and the stop codon (TGA); the 3′ non-coding region of the mRNA with a short intron and the polyadenylation signal (polyA) derived from SV40 DNA (compare to Figure 2).
b. The 7440 bp (base pairs) fragment SalI-HpaI bearing the full-length Factor VIII coding cDNA is depicted. The start and stop codons of the reading frame are indicated. Restriction endonuclease sites within the fulllength cDNA involved in the mutagenesis for the construction of pCLB202 and pCLB203 are shown.
c. Map of the Factor VIII protein. The 19 amino acid long signal peptide and the domain or repeat structure of plasma Factor VIII are shown. A1, A2 and A3 are homologous amino acid sequences. B is a unique region, whereas C1 and C2 are again homologous (Vehar et al., 1984). The amino acid positions bordering the A and C repeats are given. Below The circular plasmid pCLB201 is shown beginning with the EcoRI position within the 4217 bp HindIII-BglII fragment derived from plasmid pSV2 (Gorman, 1985 DNA Cloning (Ed. Glover) IRL Press, pp. 143-169; Mulligan and Berg, Proc. Natl. Acad. Sci. USA (1981) 78:2072). The early SV40-promoter region is indicated: SVep. The vector also contains a NdeI-HindIII fragment derived from plasmid pRSV-neo (Gorman, 1985) bearing the Rous Sarcoma Virus-Long Terminal Repeat (LTR) inserted into a SalI site. The full-length Factor VIII is encoded by a SalI-HpaI fragment of 7440 bp and indicated: Factor VIII cDNA (H = HindIII, Sal = SalI). The restriction endonuclease cleavage sites lost during construction of pCLB201 are enclosed in parenthesis. The size of the plasmid is given in kilobase pairs (kb).

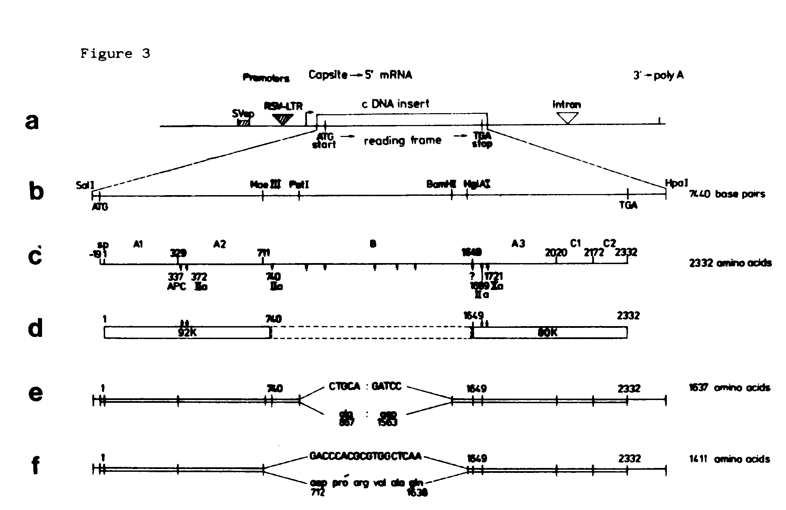

Figure 3 shows vectors for transient expression of deletion mutant Factor VIII proteins:
a. The landmarks of the pSV2-derived vector: two tandemly situated promoters: the SV40 early transcription promoter (SVep) and the Rous Sarcoma Virus-Long Terminal Repeat (RSV-LTR); the capping site (Capsite) and 5' end of the messenger RNA (mRNA); the cDNA insert bearing the full-length Factor VIII coding region with the start codon (ATG), the open reading frame and the stop codon (TGA); the 3' non-coding region of the mRNA with a short intron and the polyadenylation signal (polyA) derived from SV40 DNA (compare to Figure 1).
b. The 7440 bp (base pairs) fragment SalI-HpaI bearing the full-length Factor VIII coding cDNA is depicted. The start and stop codons of the reading frame are indicated. Restriction endonuclease sites within the full-length cDNA involved in the mutagenesis for the construction of pCLB202 and pCLB203 are shown.
c. Map of the Factor VIII protein. The 19 amino acid long signal peptide and the domain or repeat structure of plasma Factor VIII are shown. A1, A2 and A3 are homologous amino acid sequences. B is a unique region, whereas C1 and C2 are again homologous (Vehar et al., 1984). The amino acid positions bordering the A and C repeats are given. Below the map the cleavage sites of the proteolytic enzymes, that process Factor VIII, i.e., activated protein C (APC), thrombin (IIa), activated coagulation factor X (Xa), and a trypsin-like protease indicated with "?") are indicated by arrows. Factor Xa is thought to cleave the IIa- and APC-cleavage sites also shown (Eaton et al., Biochemistry (1986) 25:8343-8347; Fay et al., Biochem. Biophys. Acta (1986) 871:268-278). Their cleavage sites are given. The B region contains multiple cleavage sites.
d. The subunit structure of activated Factor VIII. Factor VIIIa subunits of 92 kDa and 80 kDa are indicated as 92k and 80k, respectively. Their amino terminal and carboxy terminal amino acid positions within the full-length sequence are indicated.
e. The structure of the cDNA and protein of pCLB202 containing a direct fusion between the PstI and BamHI site indicated in b. The resulting protein is indicated containing a peptide bond between Ala-867 and Asp-1563. The total length of the protein is 1637 amino acids. (The landmarks of the repeat border positions and specific proteolytic cleavage sites are as depicted; see above.)
f. The structure of the cDNA and protein pCLB203 containing a MluI-linker sequence coding for four extra amino acids that bridge the MaeIIIsite and HgiAT site, as indicated under b. The total length of the protein is 1411 amino acids. (The landmarks of the repeat border positions and specific proteolytic cleavage sites are depicted; see above.)

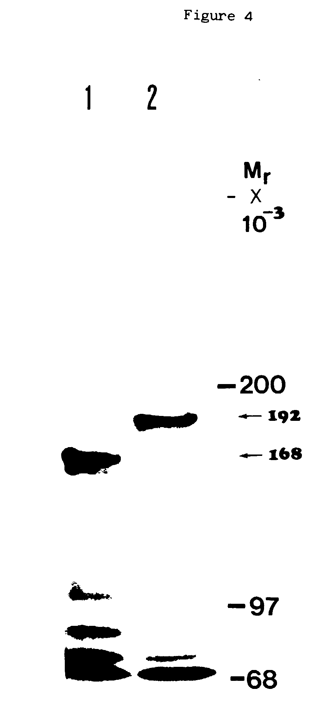

Figure 4 shows the results of a molecular weight (size) determination of several deletion mutant Factor VIII proteins using immunoprecipitation and electrophoresis.

The autoradiograph shows the lanes for the proteins made with the vectors pCLB202 (lane 2) and pCLB203 (lane 1). The molecular weight markers are as indicated.

### DESCRIPTION OF THE SPECIFIC EMBODIMENTS

In accordance with the subject invention, novel DNA constructs and novel compositions comprising host cells producing polypeptides having Factor VIII activity are provided. The polypeptides having Factor VIII activity include deletion mutant proteins of Factor VIII in which substantially all of the central region or "B domain" as well as a portion of the 92 kilo-Dalton (kDa) region has been deleted. Plasmid constructs comprising a DNA sequence encoding deletion polypeptides having Factor VIII activity are used to transform a host cell. The host cell is then grown to express the gene. The host cell may be either a eukaryotic or a prokaryotic cell.

Human Factor VIII has the sequence shown in Figure 1. Single letter abbreviations for the amino acids are used, and have the following meaning: A = alanine; R = arginine; N = asparagine; D = aspartic acid; C = cysteine; Q = glutamine; E = glutamic acid; G = glycine; H = histidine; I = isoleucine; L = leucine; K = lysine; M = methionine; F = phenylalanine; P = proline; S = serine; T = threonine; W = tryptophan; Y = tyrosine; and V = valine. The numbering of the amino acid sequence starts with A-1, the first amino acid after the 19 amino acid signal sequence. The last amino acid of Factor VIII is Y-2332. This numbering is used throughout the specification. Factor VIII-like materials, including Factor VIII fragments, mutants of the polypeptide as well as fusion peptides comprising functional portions of Factor VIII having the biological activity of the intact factor VIII, including blood clotting activity are also provided.
The polypeptides of this invention include congeners of Factor VIII, namely compounds having at least one biological activity of Factor VIII and having at least one amino acid sequence having substantially the same amino acid sequence as Factor VIII. The congener may be of greater or lesser amino acid sequence than Factor VIII. Biological activity includes the ability, when administered to patients with Hemophilia A, to correct the clotting defect and/or immunological cross-reactivity with naturally occuring Factor VIII. The correction thus may be effected either by direct action in the clotting cascade or by binding to antibodies against Factor VIII so that the biological activity of subsequently administered Factor VIII is less affected. By "immunological cross-reactivity" is meant that an antibody induced by a novel polypeptide of this invention will cross-react with intact Factor VIII. The polypeptide will have at least one biologically active sequence, for example immunological or epitopic, and may have more than one biologically active sequence, where such sequence may compete with a naturally occuring product for the biological property.

Novel polypeptides of interest will for the most part have a formula comprising an N-terminal region, N_{R}, a linking region, L_{R} and a C-terminal region, C_{R}. N_{R} is characterized as having an amino acid sequence substantially corresponding to a consecutive sequence found in amino acid sequence A-1 through R-740 of the full-length Factor VIII (the "A1A2 domain" or 92kDa polypeptide), wherein no more than 45%, usually no more than 20%, preferably no more than 10%, most preferably no more than 5% of the amino acids in the A1A2 domain have been deleted. Preferred sequences correspond substantially to the sequences A-1 through D-712 or A-1 through R-740.

L_{R} may be a short linking group of from 1 to 20 amino acids, a bond or any sequence of amino acids, usually not substantially similar to the sequence of the "B domain" of the full-length Factor VIII protein. It may also comprise sequences corresponding substantially to consecutive sequences in the B domain or any portion thereof.

C_{R} is characterized as having an amino acid sequence substantially similar to a consecutive sequence found in the sequence of Factor VIII which includes amino acids Ser-1669 through Tyr-2332, wherein no more than 25%, usually no more than 20%, preferably no more than 10% of the amino acids S-1669 through Y-2332 have been deleted. The sequence preferably includes sequences corresponding substantially to the Factor VIII sequences S-1669 through Y-2332 and S-1690 through Y-2332.

The polypeptides of interest may be fragments of Factor VIII wherein up to 75% of the amino acids have been deleted from the full length Factor VIII, or fusion proteins wherein the 92 kDa protein or a fragment thereof is fused to the 80kDa polypeptide or a fragment thereof. The polypeptide usually will have no more than about 75% of the amino acids deleted, usually no more than about 45% of the amino acids deleted, more usually no more than about 40% of the amino acids deleted.

To provide for immunogenicicity, the Factor VIII congeners can be joined covalently to a large immunogenic polypeptide entity. Exemplary of such immunogenic entities are bovine serum albumin, keyhole limpet hemocyanin (KLH) and the like. These conjugated polypeptides will be useful for inducing antibodies in an appropriate host organism. The antibodies can be used to determine the presence or absence and/or concentration of Factor VIII in a bodily fluid, the absence of which may indicate Hemophilia A.

### Preparation of Congeners of Factor VIII

Factor VIII congeners may be obtained in a variety of ways. They may be obtained by proteolytic cleavage of the full-length Factor VIII into three regions, preferably by cleavage before Arg-740 and Ser-1669 followed by truncating the amino or carboxyl terminus of the Ala1 through Pro-739 sequence at least one amino acid at a time and/or truncating the amino or carboxyl terminus of the sequence Ser-1669 through Tyr-2332 at least one amino acid at a time. The polypeptide fragments obtained may then be fused directly or via a central linking group, or the fragments may be combined in a composition to provide for Factor VIII activity.

Factor VIII congeners, including fusion proteins in which the N_{R} and C_{R} are fused, can also be prepared by recombinant DNA techniques. Techniques used in isolating the Factor VIII gene are known in the art, including synthesis, isolation from genomic DNA, preparation from cDNA, or combinations thereof. The various techniques for manipulation of the genes are well known, and include restriction, digestion, resection, ligation, in vitro mutagenesis, primer repair, and poly linkers and adapters, and the like. See Maniatis et al., Molecular Cloning, Cold Spring Harbor Laboratory, Cold Spring Harbor, New York, 1982.

Generally the method comprises preparing a genomic library from cells which synthesize Factor VIII. To enhance the likelihood of identifying the correct sequence, a cDNA library from cells which do not produce Factor VIII may be used to cross-hybridize. An assay for the expression of Factor VIII using restriction fragments inserted into a prokaryotic expression vector such as pTZ 18 or 19 and screening with antibodies for Factor VIII to detect a cross-reactive peptide fragment or the like can be used.

Once a complete gene has been identified, either as cDNA or chromosomal DNA, the desired deletions in the structural gene can be made in several ways. Deletions may be made by enzymatically cutting the full length Factor VIII cDNA followed by modification and ligation of the purified fragments or by site-directed mutagenesis, especially by loop-out mutagenesis as described by Kramer et al., Nucl. Acids Res. (1984) 12:9441-9456. The gene thus obtained may then be manipulated in a variety of ways well known in the art to provide for expression.
Both prokaryotic and eukaryotic hosts may be employed, which may include bacteria, yeast, mammalian cells, for example, CHO cells, C127 cells, human "293" cells, myeloma cells, or specialized cells such as liver cells, and COS cells. Therefore where the gene is to be expressed in a host which recognizes the wild-type transcriptional and translational regulatory regions of Factor VIII, the entire gene with its wild-type 5′, and 3′-regulatory regions may be introduced into an appropriate expression vector. Various expression vectors exist employing replication systems from mammalian viruses, such as simian virus 40, Epstein-Barr virus, bovine papilloma virus, vaccinia virus, etc.

Where the gene is to be expressed in a host which does not recognize the naturally occurring wild-type transcriptional and translational regulatory regions, further manipulation will be required. Conveniently a variety of 3'-transcriptional regulatory regions are known and may be inserted downstream from the stop codons. The non-coding 5' region upstream form the structural gene may be removed by endonuclease restriction, Bal31 resection, or the like. Alternatively, where a convenient restriction site is present near the 5′ terminus of the structural gene, the structural gene may be restricted and an adapter employed for linking the structural gene to the promoter region, where the adapter provides for the lost nucleotides of the structural gene.

Various strategies may be employed for providing a foreign expression cassette, which in the 5'-3'-direction of transcription has a transcriptional regulatory region and a translational initiation region, which may also include regulatory sequences allowing for the induction of regulation; an open reading frame encoding the full length Factor VIII or a congener of Factor VIII, including the deletion mutant proteins, desirably including a secretory leader sequence recognized by the proposed host cell; and translational and transcriptional termination regions. The expression cassette may additionally include at least one marker gene. The initiation and termination regions are functional in the host cell, and may be either homologous (derived from the original host), or heterologous (derived from a foreign source) or synthetic DNA sequences. The expression cassette thus may be wholly or partially derived from natural sources, and either wholly or partially derived from sources homologous to the host cell, or heterologous to the host cell. The various DNA constructs (DNA sequences, vectors, plasmids, expression cassettes) of the invention are isolated and/or purified, or synthesized and thus are not "naturally occurring".

For optimal gene expression, the nucleotide sequences surrounding the translational initiation codon ATG have been found to be important in animal cells. For example, Kozak, Microbiol. Reviews (1983) 47:1-45, has studied extensively the effect of these regions on the expression of polypeptides such as insulin in COS cells. Thus it may be necessary to modify the nucleotide sequences surrounding the initiation codon. This can be done by site-directed mutagenesis or by fusing the exogenous gene to the initiation region of a highly expressed gene.

Illustrative transcriptional regulatory regions or promoters include, for bacteria, the beta-gal promoter, amylase promoter, lambda left and right promoters, trp and lac promoters, trp-lac fusion promoter, etc.; for yeast, glycolytic enzyme promoters, such as ADH-1 -2 promoters, PGK promoter, and lactase promoter and the like; for mammalian cells, SV40 early and late promoters, cytomegalovirus (CMV) promoter, beta-actine promoter, adenovirus major late promoters and the like.

In eukaryotic cells, regulatory sequences can include, for example, the cytomegalovirus enhancer sequence which can be fused to a promoter sequence such as the SV40 promoter, forming a chimeric promoter, or inserted elsewhere in the expression cassette, preferably in close proximity to the promoter sequence.
Expression of the structural gene can also be amplified by, for example, ligating in tandem a gene for a dominant amplifiable genetic marker 5' or 3' to the structural gene and growing the host cells under selective conditions. An example of an amplifiable gene is the gene for dihydrofolate reductase (dhfr), the expression of which may be increased in cells rendered resistant to methotrexate (mtx), a folate antagonist. Of interest for expression in mammalian cells such as COS cells are expression cassettes capable of expressing Factor VIII or congeners thereof which employ a metallothionein promoter, or the SV40 early transcription [unit transcription] promoter (SVep), particularly the Rous Sarcoma Virus-Long Terminal Repeat (RSV-LTR) promoter in tandem with the SVep promoter. Examples of promoters and promoter/enhancer combinations which can be used in C127 cells in combination with expression vectors according to example 7 has been described in e.g. Hurwitz et al., Nucl. Acids Res. (1987) 15:7137-7153. For optimal expression the introduction of an intron into the expression cassette, may be beneficial. An intron in the 5′-part of the mRNA is preferred.

In addition, a fused gene may be prepared by providing a 5′-sequence to the structural gene which encodes a secretory leader and a processing signal. For secretion of Factor VIII polypeptides the naturally occurring Factor VIII leader sequence is preferably used, but other signal sequences including secretory leaders of penicillinase, alfa-factor, immunoglobulin, T-cell receptors, outer membrane proteins, serum albumin, tissue plasminogen activator, insulin, digestive tract enzymes, and the like may also be used. By fusion of a secretory leader in proper reading frame with a structural gene for Factor VIII or a congener therof, the mature Factor VIII may be secreted into the culture medium.

The termination region may be derived from the 3′ region of the gene from which the initiation region was obtained or from a different gene. A large number of termination regions are known and have been found to be satisfactory in a variety of hosts from the same and different genera and species. The termination region may include sequences for the proper processing of the mRNA in a mammalian cell: i.e., a small intron and a polyadenylation signal.

During the construction of the expression cassette, the various DNA fragments will usually be cloned in an appropriate cloning vector, which allows for expansion of the DNA, modification of the DNA or manipulation by joining or removing of the sequences, linkers, or the like. Normally, the vectors will be capable of replication in at least a relatively high copy number in E. coli. A number of vectors are readily available for cloning, including such vectors as pBR322, pML2, pUC7-pUC19, pSP64, pSP65, pSP18, pSP19, pTZ18 and pTZ18R, pTZ19 and pTZ19R and the like.

The cloning vectors are characterized as having an efficient origin of replication at least functional in E. coli. Also the cloning vector will have at least one unique restriction site, usually a plurality of unique restriction sites and may also include multiple restriction sites, particularly two of the same restriction sites for substitution. In addition, the cloning vector will have one or more markers which provide for selection for transformation. The markers will normally provide for resistance to cytotoxic agents such as antibiotics, heavy metals, toxins or the like, complementation of an auxotrophic host, or immunity to a phage. By appropriate restriction of the vector and cassette, and, as appropriate, modification of the ends, by chewing back or filling in overhangs, to provide for blunt ends, by addition linkers, by tailing, complementary ends can be provided for ligation, and joining of the vector to the expression cassette or a component thereof.

In some instances, a shuttle vector will be employed where the vector is capable of replication in different hosts requiring different replication systems. Preferably a simian virus 40 (SV40) origin of replication is employed which allows the plasmid to be propagated in COS-1, while the Bovine Papilloma Virus (BPV-1) genome is used for the episomal maintenance of expression vectors in C127 cells (see for example Howley et al., Meth. Enzymol. 101: 387-402, 1983).

The expression cassette may be included within a replication system for episomal maintenance in an appropriate cellular host or may be provided without a replication system, where it may become integrated into the host genome. The manner of transformation of the host organism with the various DNA constructs is not critical to this invention. The DNA may be introduced into the host in accordance with known techniques, such as transformation, using calcium phosphate precipitated DNA, electroporation, transfection by contacting the cells with a virus, microinjection of the DNA into cells or the like.

As a host organism, normal primary cells or cell lines derived from cultured primary tissue may be used, as well as microbial cells. The host cell is preferably a mammalian cell line, preferably hamster CHO cells, mouse C127 cells or human "293" cells, but microbial cells, for example yeast, preferably a Kluyveromyces species, or bacteria, preferably a Bacillus species, may also be used.

For stable transformation of a mammalian cell line with the expression vector bearing the Factor VIII congener coding sequence and subsequent amplification of the vector inserted in the host chromosome, Chinese hamster ovary cells (CHO) are especially suitable. Transformation involves transfection of the host cell with the expression vector together with a selectable marker gene such as dhfr or a G418 (neo-) resistance gene or the like, and subsequent integration into the chromosome and selection for stable transformation.

Another method for stable transformation for host cell lines uses expression vectors containing BPV-1 sequences. C127 cells are well suited for this purpose. Transformation involves transfection of the host cell with the expression vector containing BPV-1 sequences. Transformants are recognized by the formation of foci. Stable transformants are established and screened for Factor VIII activity using the Kabi Coatest according to examples 6 and 7. In contrast, when the expression is carried out in a transient transformation system such as COS-1 cells with pSV2-derived expression vectors, there is no selection step. Once the structural gene has been introduced into the appropriate host, the host may be grown to express the structural gene. The host cell may be grown to high density in an appropriate medium. Where the promoter is inducible, such as in a prokaryotic system, permissive conditions will then be employed, for example temperature change, exhaustion, or excess of a metabolic product or nutrient, or the like. In a mammalian system, where an amplifiable gene is used in tandem with the structural gene, the appropriate means for amplification will be employed.

Where secretion is provided for, the expression product, either fused or unfused, may be isolated from the growth medium by conventional means. Where secretion is not provided for, the host cells may be harvested and lysed in accordance with conventional methode. The desired product is then isolated and purified by conventional techniques, for example affinity chromatography with immobilized antibodies, chromatography on aminohexyl-sepharose or the mixed polyelectrolyte method.

The recombinant products may be glycosylated or non-glycosylated, having the wild-type or other glycosylation. The amount of glycosylation will depend in part upon the sequence of the particular peptide, as well as the organism in which it is produced. Thus, expression of the product in E. coli cells will result in an unglycosylated product, and expression of the product in mammalian cells will result in a product with glycosylation similar to the wild-type peptide.

### Uses of Factor VIII Congeners

The subject compounds can be used in a wide variety of ways, both in vivo and vitro. The subject compounds may be used as the active component of pharmaceutical preparations for treating patients exhibiting symptoms of Hemophilia A. By a pharmaceutical composition is meant any preparation to be administered to mammals. Thus, a pharmaceutical preparation with Factor VIII activity is a preparation which can be administered to a mammal to alleviate symptoms associated with Hemophilia A, the inability to properly clot blood. In preparing the pharmaceutical composition, generally the subject polypeptides are admixed with parenterally acceptable vehicles and other suitable excipients in accordance with procedures known in the art. The pharmaceutical preparations may conveniently be a sterile lyophilized preparation of the subject polypeptide which may be reconstituted by addition of a sterile solution suitable for producing solutions, preferably isotonic with the blood of the recipient. The pharmaceutical preparation may be presented in single unit or multi-dose containers, for example in sealed ampoules or vials. Their use would be analogous to that of known human Factor VIII polypeptide, appropriately adjusted for potency. The subject polypeptide may be administered in vivo, for example by injection, intravenously, peritoneally, subcutaneously, or the like.

The subject compounds additionally can be used for making antibodies to the subject compounds, which may find use in vivo or in vitro. The antibodies can be prepared in conventional ways, either by using the subject polypeptide as an immunogen and injecting the polypeptide into a mammalian host, for example mouse, cow, goat, sheep, rabbit, etc., particularly with an adjuvant, for example complete Freunds adjuvant, aluminum hydroxide gel, or the like. The host may then be bled and the blood employed for isolation of polyclonal antibodies, or in the cases of the mouse, the peripheral blood lymphocytes or splenic lymphocytes (B cells) employed for fusion with an appropriate myeloma cell to immortalize the chromosomes for monoclonal expression of antibodies specific for the subject compounds.

Either polyclonal or monoclonal antibodies may be prepared, which may then be used for diagnosis or detection of the presence of the subject polypeptide in a sample, such as cells or a physiological fluid, for example blood. Failure to detect the subject polypeptide may indicate the condition of hemophilia A.

Probes comprising sequences complementary to Factor VIII mRNA may also be prepared and used as a diagnostic aid, for example, the presence and/or amount of Factor VIII mRNA in a cell may be used for determining whether the patient is making Factor VIII but has developed antibodies which prevent its activity. A test sample comprising a cell, tissue sample or bodily fluid believed to contain hybridizing sequences can be treated so as to lyse any cells, then treated with a denaturing agent such as guanidine hydrochloride to release single-stranded mRNA. The probe labeled with, for example ³²P or biotinylated nucleotides, can then be hybridized to the cellular mRNA to form a double-stranded complex which may be detected by means of the label. For some purposes it may be desirable to quantitate the amount of Factor VIII mRNA. This is done by comparing the amount of label detected in reference samples containing known amounts of single-stranded Factor VIII mRNA with the amount of label detected in the test sample.

The following examples are offered by way of illustration and not by way of limitation.

### EXPERIMENTAL

General cloning techniques were used as described in Maniatis et al., Molecular Cloning: A Laboratory Manual, Cold Spring Harbor Laboratory, CSH, NY, 1982. All DNA-modifying enzymes were obtained from commercial suppliers. They were used according to manufacturers instructions. Materials and apparatus for DNA purification and separation were used according to instructions from the supplier.

### Example 1

### Construction of Expression Vector PCLB201

pCLB201 is an expression vector comprising the RSV-LTR promoter in the right orientation for transcriptional initiation, and the full-length Factor VIII cDNA (see Figure 2). It is derived from the pSV2-vector of Mulligan and Berg, Proc. Natl. Acad. Sci. USA (1981) 78:2072.

The unique HindIII site of the plasmid pSV2.tPA (Van Zonneveld et al., Proc. Natl. Acad. Sci. USA (1986) 83:4670-4674) was modified into a SalI site by making blunt the HindIII sticky-ends and ligating SalI-linkers (5′-CGTCGACG-3′) to the blunt ends. A plasmid containing a SalI site was selected and identified as pCLB91. This plasmid is the same as pSV2.tPA except that it contains a SalI linker inserted into the HindIII site.

The isolation of Factor VIII mRNA from human liver, and the preparation, purification and identification of its cDNA and its assembly in the plasmid pEP121 resulting in plasmid pCLB89 have been described in patent application EP 0253455, which disclosure is hereby incorporated by reference. A 7440 bp long SalI-HpaI fragment from pCLB89 containing the factor VIII cDNA (see Figure 1) was purified and inserted into pCLB91 digested with SalI and BglII. The resulting expression vector pCLB200 contained an intact SalI site with the HpaI site ligated to the BglII site which had been filled in.

Plasmid pRSVneo (Gorman, DNA Cloning, 1985, ed., D. Glover, IRL-press, Washington, Oxford, pp. 143-169) was digested with NdeI and HindIII and incubated with the Klenow fragment of DNA polymerase to create blunt ends and the 0.6 kb fragment containing a Rous Sarcoma Virus-Long Terminal Repeat (RSV-LTR) sequence was isolated and inserted into the SalI site of pCLB200 which had similarly been made blunt-ended to form expression vector pCLB201 (see Figure 2).

### Example 2

### Construction of pCLB202

A deletion mutant of Factor VIII having a deletion from the PstI site at nucleotide sequence position 2660 (see Figure 3b) to the BamHI site at postition 4749 (see Figure 3b) was constructed in plasmid pGB860 as described in patent application EP 0253455. The Factor VIII DNA in pGB860 has a deletion of the DNA coding for 695 amino acids in the central region.

Expression vector pCLB202 was derived from pGB860 and pCLB201. Both plasmids were digested with KpnI and XbaI restriction enzymes. The 3.1 kb KpnI-XbaI fragment in pCG860 was ligated to the 7 kb KpnI-XbaI fragment from pCLB201. The resulting plasmid pCLB202 has intact KpnI and XbaI sites. Its structure is depicted in Figure 3e.

### Example 3

### Construction of pCLB203

### (A) pCLB100:

The SalI-HpaI 7440 bp fragment (see Figure 2) from pCLB89 was inserted into plasmid pSP64 (Melton et al., Nucleic Acids Res. (1984) 12:7035-7056) which had been cleaved with SalI and SmaI. The resulting plasmid, pCLB100, contained an intact SalI-site and the HpaI-end linked to the SmaI-end.

### (B) pCLB101: (numbers in parentheses refer to Figure 1)

(1) Plasmid pCLB100 was digested with KpnI and
   TthlllI, generating a 631 bp fragment: KpnI (1817) to TthlllI (2447). The 631 bp fragment was purified, then cut with MaeIII (2199). The MaeIII sticky end was filled in resulting in a 383 bp fragment.
(2) Plasmid pCLB100 was digested with ApaI and
   BanI. The 669 bp ApaI (6200)-BanI (5532) fragment was isolated.
(3) Plasmid pCLB100 was digested with HgiAI,
   producing a HgiAI-fragment of 634 bp. The fragment incubated with T4 DNA polymerase to make blunt ends, then digested with BanI, to produce a 565 bp fragment.
(4) Plasmid pCLB100 was digested with ApaI and
   KpnI, generating a 5,9 kb long fragment (ApaI (6200) to KpnI (1817)) containing vector sequences for maintenance and propagation in E. coli.
(5) The fragments obtained in steps (1) through (4) were purified and equimolar amounts of the fragments and a ten-fold molar excess of MluI-linker (CCACGCGTGG) were ligated, giving rise to plasmid pCLB101. Plasmid pCLB101 contained a MluI-linker between the MaeIII and HgiAI sites (see Figure 3b) in addition to KpnI, BanI and ApaI sites. Four extra amino acids (P-R-V-A) between amino acids D-712 and Q-1638 (Figure 1) are shown in Figure 3f.

### (C) pCLB203:

pCLB101 and pCLB201 were digested with enzymes KpnI and XbaI. The 2.4 kb KpnI-XbaI fragment from pCLB101 was ligated to the 7.0 kb KpnI-XbaI fragment derived from pCLB200. The resulting plasmid pCLB203 possessed intact KpnI and XbaI sites. Its structure is depicted in Figure 3f.

### Example 4

### Construction of pCLB212

pCLB212 was constructed using the loop-out mutagenesis technique as described in Kramer et al., (1984) Nucleic Acids Res. 12:9441-9456. The deletion mutagenesis of the central region of Factor VIII cDNA was performed using the following oligonucleotide:

### Primer IV

3' TTC.CAA.AGA.TCA.ACA.CTG.GTT.TTG.GGT.GGT.CAG.AAC 5'

to produce in factor VIII cDNA internal deletion of the sequences coding for amino acids Lys 713 through Ser-1637.

The corresponding protein contains an internal deletion of 925 amino acid residues compared with the full length Factor VIII protein.

To obtain a target fragment for loop-out mutagenesis a HindIII-PstI fragment of 1.4 kb derived from pCLB203 (Example 3) was selected. The nucleotide position (Figure 1) of the HindIII-site is at 1025 in the full length Factor VIII sequence upstream of the region to be modified, the PstI site is at position 5165 downstream of the region. These sites are indicated in Figure 2. The 1.4 kb fragment was subcloned in M13mp9 followed by the loop-out mutagenesis. After selecting the fragment with the precise deletion, this was followed by the insertion of the KpnI-PstI part of the HindIII-PstI fragment containing the desired deletion into an appropriate expression vector by preparing fragments with sticky, unique restriction enzyme ends (numbers refer to Figure 1), according to the following steps:

Step 1. For the expression of the mutant Factor VIII molecules a new plasmid pCLB211 was constructed. The SalI-HpaI fragment of 7440 bp (Figure 1) derived from pCLB89 was inserted into the expression vector pSVL (Pharmacia, No. 27-4509-01, Uppsala, Sweden) enabling transcription from a late SV40 promoter in mammalian cells. The plasmid pSVL was cleaved with XhoI and SmaI. The resulting plasmid pCLB211 contains an XhoI end linked to the SalI end, since both 5′ protruding ends of these enzymes are identical, and the SmaI end linked to the HpaI end.

Step 2. Plasmid pCLB211 was digested with ApaI and SalI. The 1.4 kb ApaI(6200)-SalI(unique in pSVL-part of pCLB211) fragment was isolated.

Step 3. Plasmid pCLB100 was digested with NdeI, PstI and ApaI. Two fragments were isolated: a PstI(5165)-NdeI(5527) fragment of 363 bp and second fragment, NdeI(5527)-ApaI(6200) of 674 bp.

Step 4. Plasmid pCLB100 was digested with KpnI and SacI. A KpnI(1817)-SacI(19) of 1799 bp was isolated.

Step 5. Plasmid pCLB211 was digested with SalI and SacI. A 4.5 kb SalI(unique in the pSVL vector)-SacI(19) fragment was obtained.

Step 6. An M13-bacteriophage containing a HindIII(1025)-PstI(5165) fragment with the desired deletion as verified with sequence analysis, was digested with KpnI and PstI. A 584 bp PstI(5165)-KpnI(1819) fragment was isolated.

Step 7. The six isolated fragments from Steps 2 through 6 were mixed in equimolar amounts and ligated. A plasmid containing all six fragments was selected. The plasmid pCLB212 expressed the exemplary compound 3b (Table II). The compound 3b differs from compound 3 (illustrated in Figure 3f) as it is missing the MluI linker and corresponding four amino acids.

### Example 5

### Construction of pCLB208, pCLB209 and pCLB210

pCLB208, pCLB209 and pCLB210 were contructed using the loop-out mutagenesis technique as described in Kramer et al., Nucleic Acids Res. (1984) 12:9441-9456.

### (a) Loop out mutagenesis

Deletion mutagenesis of the central region of Factor VIII cDNA was performed using the following oligonucleotides:

### Primer I:

3' TTA.CGG.TAA.CTT.GGT.TCT.CTT.TAT.TGA.GCA.TGA.TGA 5'

### Primer II:

3' TTA.CGG.TAA.CTT.GGT.TCT.AGT.CAA.CTT.TAC.TTC.TTC 5'

### Primer III:

3' TTA.CGG.TAA.CTT.GGT.TCT.TCG.AAA.GTT.TTC.TTT.TGT 5'

to produce internal deletions in Factor VIII cDNA of the sequences coding for amino acids S-741 through R-1648 (Primer I), S-741 through I-1668 (Primer II) and S-741 through R-1689 (Primer III). The corresponding proteins contain internal deletions of 908 (I), 928 (II) and 949 (III) amino acid residues, respectively.

### B. Preparation of Target Fragments

To obtain a target fragment for loop-out mutagenesis a fragment of 0.8 kb derived from pCLB101 was constructed as follows.

Plasmid pCLB101 was digested with EcoRI, the EcoRI sites were filled in and a further digestion with KpnI followed. A 479 bp KpnI(1819)-EcoRI(2297) fragment (the numbers of nucleotide positions are as described in Figure 1) was isolated. Plasmid pCLB100 was digested with BamHI; the sticky ends were filled in and a further digestion with PstI followed. A 416 bp BamHI(4749)-PstI(5165) fragment was isolated. These fragments were ligated in equimolar amounts and subcloned in M13mp19 amber. The M13 bacteriophage containing a KpnI-PstI fragment of 895 bp from 1819 upstream from the region to be modified to 5165 downstream from that region and with a large deletion in the Factor VIII coding sequence was selected for the loop out mutagenesis. After selecting the fragment with the precise deletion obtained with Primer I, II, or III in bacteriophage M13, the KpnI-PstI fragment containing the desired deletion was inserted into an appropriate expression vector derived from pCLB211 (see Example 4) using the following steps and preparing fragments with sticky, unique restriction enzyme ends (numbers refer to Figure 1):

(1). Plasmid pCLB211 was digested with ApaI and SalI. The 1.4 kb ApaI(6200)-SalI (unique in pSVL-part of pCLB211) fragment was isolated.

(2). From plasmid pCLB100 digested with NdeI, PstI and ApaI a 363 bp PstI(5165)-NdeI(5527) fragment and a 674 bp NdeI(5527)-ApaI(6200) fragment were isolated.

(3). Plasmid pCLB100 was digested with KpnI and SacI and a 1799 bp KpnI(1817)-SacI(19) fragment isolated.

(4). Plasmid pCLB211 was digested with SalI and SacI. The 4.5 kb SalI(unique in the pSVL vector)-SacI(19) fragment was isolated.

(5). The M13-bacteriophage containing the fragment with the desired mutation was digested with KpnI and PstI. The KpnI-PstI fragments containing the desired mutation were isolated for all three mutageneses.

(6). The six fragments from (1) to (5) above were isolated, mixed in equimolar amounts and ligated. Plasmids containing all of the desired fragments were selected by hybridization and restriction enzyme digestion. Using Primers I, II or III shown above, three new expression vectors were constructed for the expression of the exemplary compound described in Table I:
1. pCLB208 for compound 7 with Primer I;
2. pCLB209 for compound 8 with Primer II; and
3. pCLB210 for compound 9 with Primer III.

### Example 6

### Transient Expression of Recombinant Factor VIII DNA and Assay of Produced Proteins

### A. Transfection of COS-1 Cells and metabolic labeling

The expression vectors obtained according to the previous examples were introduced into COS-1 cells using a DEAE transfection technique. Vector DNA was precipitated by incubating with DEAE-dextran for 2 hrs, followed by treatment with chloroquine shock for 2 hrs, as described by Lopata et al., Nucleic Acids Res. 12, 5707, (1984) and Luthman and Magnusson, Nucleic Acids Res. (1983) 11:1295. The medium used for growth of the COS-1 cells and also for the conditioned medium was Iscove's DMEM (Flow) supplemented with 10% (v/v) heat inactivated fetal calf serum (Flow). The medium was changed 48 hrs after transfection. The conditioned medium was collected 48 hrs later.

Metabolic labeling of the proteins in the transfected cells was carried out using serum free RPMI-medium (Gibco). Two days after transfection transfectants were incubated for 4 hrs with 50 uCi/ml L-³⁵S-methionine (Amersham, 1985; 370 MBeq/330 ul; specific activity: over 100 Ci/mMole), followed by an overnight incubation with 1 mM L-methionine before harvesting the conditioned medium. In order to suppress protein degradation, protease inhibitors such as phenyl methane sulphonyl fluoride (PMSF) were added to the conditioned medium after harvesting. To inhibit protein glycosylation, tunicamycin can be added to the conditioned medium (final concentration 0.001 mg/ml). Conditioned media were harvested 4 days after transfection; produced proteins were assayed.

### B. Biological Activity of Recombinant Factor VIII

The conditioned medium was assayed for Factor VIII activity using (1) the standard coagulation or clotting assay and (2) the chromogenic activity assay (Kabi Coatest).

The standard coagulation or clotting assay (so-called activated partial thromboplastin time) was performed using hemophilia plasma as described by Veldkamp et al., Thromb. Diath. Haemorrh. (1968) 19:279. The conditioned medium was citrated before examination.

The chromogenic activity or Kabi Coatest assay was performed according to procedures supplied by the manufacturer Kabi-Vitrum, except that all volumes prescribed were divided by four and 25 ul of conditioned medium was tested. The Factor VIII-like proteins were activated for 15 min, at which time the chromogenic substrate (S2222) was added.

Inhibition of Factor VIII activity was measured according to the standard Bethesda protocol (Kasper et al., Thromb. Diath. Haemorrh. (1975) 34:869-871. The immunoglobulins used were purified by ion exchange and protein A Sepharose chromatography prior to use.

The standard for biological activity assays for Factor VIII was a pool of citrated plasma (0.5 mM final conc.) which was assumed to contain 1 U of Factor VIII activity or antigen per ml.

The biological activity for various of the deletion proteins are shown in Table II.
Mutant proteins with deletions exhibited Factor VIII clotting activity.

In addition, the established activity of the recombinant protein solution appeared to be inhibited following addition of antibodies known to be inhibitors of plasma-derived Factor VIII activity and/or of so-called inhibitor-sera, obtained from patients with inhibitors, i.e., antibodies against Factor VIII, in their blood.

### C. Immunologic Cross-reactivity with Factor VIII

### (1) Preparation of monoclonal antibodies.

Balb/c mice were immunized with purified human Factor VIII-von Willebrand factor complex obtained by agarose gel filtration of cryoprecipitate of a Factor VIII concentrate used for therapeutical purposes (Central Laboratory of the Netherlands Red Cross Blood Transfusion Service, Amsterdam, The Netherlands), Van Mourik and Mochtar, Biochim. Biophys. Acta 221:677-679 (1970). Lymphocyte hybridization was performed as described by Galfre et al., Nature (1977) 266:550-552. Description of the techniques used for selection of clones producing monoclonal antibodies to Factor VIII has been provided elsewhere (Stel, 1984, Ph.D. thesis, University of Amsterdam, The Netherlands). Monoclonal antibodies to Factor VIII were identified according to their reactivity with Factor VIII polypeptides as described in European patent application EP 0253455.

### (2) Polyclonal antibodies to Factor VIII polypeptides.

Rabbits were immunized by standard procedures with a Factor VIII preparation which had been purified by chromatography (Stel, supra). The antibodies thus obtained were identified by immunoblotting, as described in European patent application EP 0253455, using purified Factor VIII-von Willebrand factor or purified polypeptides. The antibodies were isolated by polyacrylamide gel electrophoresis then transferred to nitrocellulose sheets as target proteins. Three distinct polyclonal antisera were obtained: RH 63271, RH 63272 and RH 63275. The antisera reacted with the 80 kDa doublet, 92 kDa polypeptide and larger polypeptides. These antisera also reacted with smaller fragments of Factor VIII.

### (3) ELISA

A newly developed ELISA (Enzyme Linked Immuno-Sorbent Assay) was used to detect Factor VIII antigen in the conditioned medium. The method is as follows. The ELISA plates were coated by adding 200 ul/well of an appropriate dilution of the Factor VIII specific monoclonal antibody CLB.CAgA (5 mg/l) in 0.05 M carbonate buffer, pH 9.8. The plates were sealed and incubated overnight at 4°C. Between all incubations the plates were washed three times with PBS containing 0.05% Tween-20, which was left on the plates for at least one minute.

A series of dilutions of test samples or normal plasma was pipetted into the wells (200 ul/well) in duplicate. The plates were sealed and incubated at 37°C for 2 hrs without stirring, then washed as described above. For dilution of the antigen a buffer was used which contained 50 mM Tris-HCl buffer, 2% bovine serum albumin and 1.0 M sodium chloride, pH 7.4.

CLB-CAg117-horse radish peroxidase conjugates were diluted approximately 10,000-fold (depending on the sensitivity required) in a buffer containing 50 mM Tris-HCl buffer, 0.2% Tween-20 and 1.0 M sodium chloride, pH 7.4. Of this dilution, 200 ul was added to each well, the plates were sealed and incubated for 2 hrs at 37°C in the dark.

After washing the plates as described above, 150 ul of a freshly prepared solution of tetramethylbenzidine (TMB) (0.1 g/l) and hydrogen peroxide (0.006%) in 0.1 M acetate/citric acid buffer, pH 5.5 was added to each well. The plates were incubated for 30 min in the dark at room temperature. The enzyme reaction was stopped by the addition of 150 ul 2N sulphuric acid. Adsorption was determined at 450 nm with an ELISA microreader. As shown in Table II there was an increase in Factor VIII activity of successive conditioned medium which was approximately proportional to the increase in the amount of Factor VIII proteins in the medium.

### D. Size determination

The size of the produced Factor VIII proteins was established using gel electrophoresis as follows.

The monoclonal and polyclonal sera raised against plasma-derived Factor VIII were used for immunoprecipitation. The antibodies were immobilized on protein A-Sepharose (15 ul serum per 10 mg protein A-Sepharose), then incubated with the metabolically labeled recombinant Factor VIII-like compounds. The immobilized recombinant proteins were reduced using 10% beta-mercaptoethanol, then separated on a 5% polyacrylamide SDS gel electrophoresis system (Laemmli, Nature (1972) 227:680-685). As shown in Figure 4 and Table III, the recombinant Factor VIII-like proteins have the size expected for glycosylated proteins. The smaller bands were also present in the control lane.

Based upon the results presented in Table III it can also be concluded that the recombinant Factor VIII-like proteins of the subject invention are glycosylated, since there is a significant difference between the size of the proteins formed in medium with and without tunicamycin, a known inhibitor of asparagine-linked glycosylation.

### Example 7

### Construction of Stable Cell Lines Producing Proteins with Factor VIII Activity

### A. Expression in CHO Cells

Plasmids pCLB203 (10 ug) and pAdD26SV(A)-3 (1 ug, Kaufman and Sharp, Mol. Cell Biol. (1982) 2:1304-1319) were introduced into dhfr-deficient CHO cells (Chasin and Urlaub, Proc. Natl. Acad. Sci. USA (1980) 77:4216-4220) using the calcium phosphate precipitation technique (Graham and Van der Eb, Virology (1973) 52:456-467). Amplification of Factor VIII and dhfr coding sequences was achieved by stepwise administration of increasing concentrations of methotrexate (mtx) as described by Kaufman and Sharp, J. Mol. Biol. (1982) 159:601-621. Independent transformants resistant to 200 nM mtx were picked and established as stable cell lines. Several of these cell lines produced about 75 mU of Factor VIII activity/ml of culture medium. The amount of Factor VIII activity secreted into the culture medium could be further raised by further amplification of the Factor VIII coding sequences, using mtx in increasing concentrations.

### B. Expression in C127 Cells

As described above, expression vectors may be introduced into eukaryotie cells, where they integrate into the genome of the host cell. Subsequently the expression cassettes may be amplified. An alternative to integration of the expression vector is its stable maintenance as an episome. An example of the latter is the expression of one of the "mutant" Factor VIII protein using the episomal BPV-system (Howley et al., Meth. Enzymol. (1983) 101:387-402). The BPV-1 genome (BamHI cleaved) was first introduced into a BamHI cleaved derivative of pTZ18R (Pharmacia) which contains XhoI sites on both sites of the BamHI site. Subsequently the resulting p TZX-BPV plasmid was cleaved using XhoI, yielding a 2.9 kb pTZ fragment and an 8 kb BPV-fragment with XhoI protruding ends. The latter fragment was ligated into plasmid pCLB212 which had been cleaved at its unique SalI site (at position 2040 in the original pSVL vector). The resulting expression vector pGB881 contains the SV40 late promoter, Factor VIII cDNA coding sequence lacking 2775 bp (mainly of the B-domain) and SV40 late polyadenylation signal. Due to the presence of the BPV-genome this vector can be maintained as an episome in the proper host cells, such as mouse C127 cells. Plasmid pGB881 (10 ug) was transfected into C127 cells using the calcium phosphate precipitation technique. (Graham and Van der Eb, supra). Foci were isolated 14 days after transfection and subsequently stable cell lines were established. Several cell lines produced 40 mU of Factor VIII activity/ml of culture medium.

### Example 8

### CONSTRUCTION OF pCLB204.

pCLB204 was constructed using the loop-out mutagenesis technique as described in Kramer et al. (1984) (see General Methods, 2). The deletion mutagenesis of the central region of factor VIII cDNA was performed using the following oligonucleotide:

### primer V:

3' TAG.GTT.TAA.GCG.AGT.CAA.GTT.TTC.GGT.GGT.CAG.AAC 5'

to produce in factor VIII cDNA internal deletions of the sequences coding for amino acids Ala-375 through Ser-1637 (primer V).

The corresponding protein contains internal deletions of 1263 amino acids.

To obtain a target fragment for loop-out mutagenesis a HindIII-PstI fragment of 1.4 kb derived from pCLB203 (Example 3) was selected. The nucleotide position (Figure 1) of the Hind III-site is at 1025 in the full length factor VIII sequence upstream of the region to be modified; the PstI site is at position 5165 downstream of the region. These sites are indicated in Fig. 2. The 1.4 kb fragment was subcloned in M13mp9 followed by the loop-out mutagenesis. After selecting the fragment with the precise deletion obtained with primer V in bacteriophage M13, the HindIII-PstI fragment containing the desired deletion was inserted into an appropriate expression vector derived from pCLB201, using the following steps and preparing fragments with sticky, unique restriction enzyme ends (numbers refer to Figure 1):

step 1. Plasmid pCLB201 was digested with AvaI and XbaI, generating a vector fragment - AvaI (737) till XbaI (6951) - of about 6 kb.

step 2. Plasmid pCLB201 digested with AvaI and HindIII gave a 289 bp long fragment: AvaI (737) till HindIII (1025).

step 3. Plasmid pCLB201 digested with PstI and NdeI gave a fragment - PstI (5165) till NdeI (5527) - of 363 bp.

step 4. Plasmid pCLB201 digested with NdeI and XbaI gave a fragment - NdeI (5527) till XbaI (6951) - of 1425 bp.

step 5. The M13 bacteriophage containing the fragment with the desired mutation was digested with HindIII and PstI.

step 6. The five fragments were isolated, mixed in equimolar amounts and ligated. Plasmids containing all fragments were selected. Using primer V, shown above, a new expression vector was constructed for the expression of the examplary compound described in Table I:
pCLB204 for compound 4 with primer V

The DNA constructs and methods of the present invention provide a means for preparing polypeptides having Factor VIII activity by introducing a deletion mutant gene encoding a Factor VIII congener into a host cell. The subject compositions find use for treatment of the symptoms of Hemophilia A.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

### REFERENCES

Brinkhous, K.M. et al., (1985)
Proc. Natl. Acad. Sci. USA 82, 8752-8756

Burke, R.L. et al., (1986)
J. Biol. Chem. 261, 12574-12578.

Carter, P. et al., (1985)
Nucleic Acids Res. 13, 4431

Chasin, L.A. and Urlaub, G. (1980)
Proc. Natl. Acad. Sci. USA 77, 4216-4220

Eaton, D.L. et al., (1986a)
Biochemistry 25, 505-512

Eaton, D.L. et al., (1986b)
Biochemistry 25, 8343-8347

Fay, J.F. et al., (1986)
Biochim. Biophys. Acta 871, 268-278

Galfré, G. et al., (1977)
Nature 266, 550-552

Gluzman, Y., (1981)
Cell 23, 175-182

Gorman, C., (1985)
DNA cloning (Ed.: D. Glover), IRL-press, Washington, Oxford, pp. 143-169

Graham, F. and Van der Eb, A. (1973)
Virology 52, 456-467

Hanahan, D., (1983)
J. Mol. Biol. 166, 557-580

Howley, P.M., Sarver, N. and Law, M.F. (1983)
Meth. Enzymol. 101, 387-402

Hurwitz, D.R., Hodges, R., Drohan, W. and Sarver, H. (1987)
Nucl. Acids Res. 15, 7137-7153

Kasper, C.K. et al., (1975)
Thrombs. Diath. Haemorrh. 34, 869-871

Kaufman, R.J. and Sharp, P.A. (1982)
Mol. Cell. Biol. 2, 1304-1319

Kaufman, R.J. and Sharp, P.A. (1982a)
J. Mol. Biol. 159, 601-621

Kozak, M. (1983)
Microbiol. Rev. 47, 1-45

Kramer, W. et al., (1984)
Nucleic Acids Res. 12, 9441-9456

Laemmli, U.K., (1970)
Nature 227, 680-685

Luthman, H. and Magnusson, G. (1983)
Nucleic Acids Res. 11, 1295

Maniatis, T. et al., (1982)
Molecular Cloning. Cold Spring Harbor Laboratory

Maxam, A.M. amd Gilbert, W. (1980)
Methods Enzymol. 65, 499-560

Melton, D.A. et al., (1984)
Nucleic Acids Res. 12, 7035-7056

Mulligan, R. and Berg, P. (1981)
Proc. Natl. Acad. Sci. USA, 78, 2072

Sanger, F. et al., (1977)
Proc. Natl. Acad. Sci. USA 74, 5463-5467

Stel, H.V., (1984)
Ph.D. thesis, University of Amsterdam, The Netherlands

Toole, J.T., et al., (1986)
Proc. Natl. Acad. Sci. USA 83, 5939-5942

Van Mourik, J.A. and Mochtar, J.A., (1970)
Biochim. Biophys. Acta 221, 677-679

Van Zonneveld, A.J., et al., (1986)
Proc. Natl. Acad. Sci USA 83, 4670-4674

Vehar, G.A., et al., (1984)
Nature 312, 337-342

Veldkamp, J.J. et al., (1968)
Thromb. Diathes Haemorrh. 19, 279

Wood, W.I. et al., (1984)
Nature 312, 330-337

Yanisch-Perron, C. et al., (1985)
Gene 33, 103-119

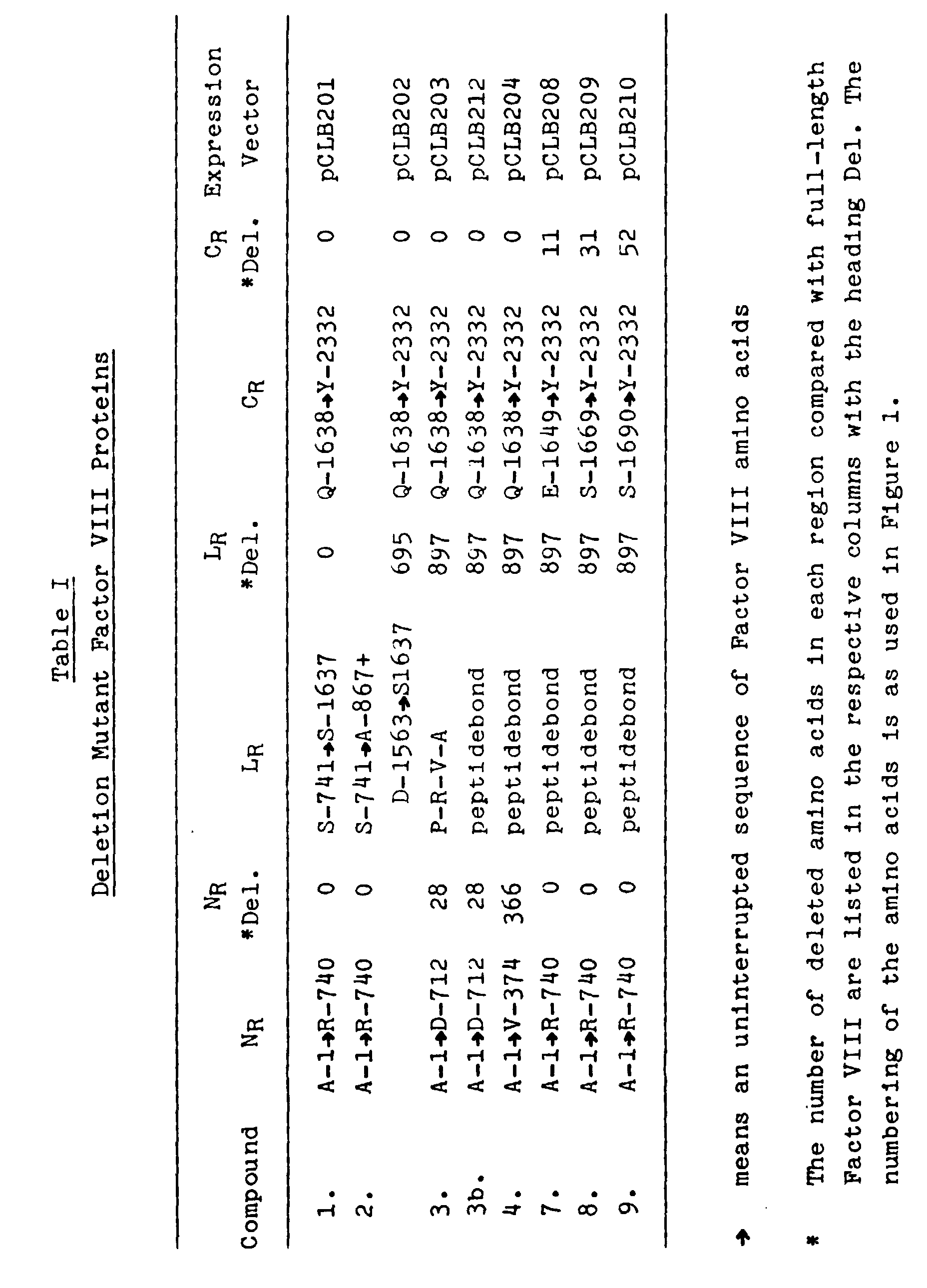

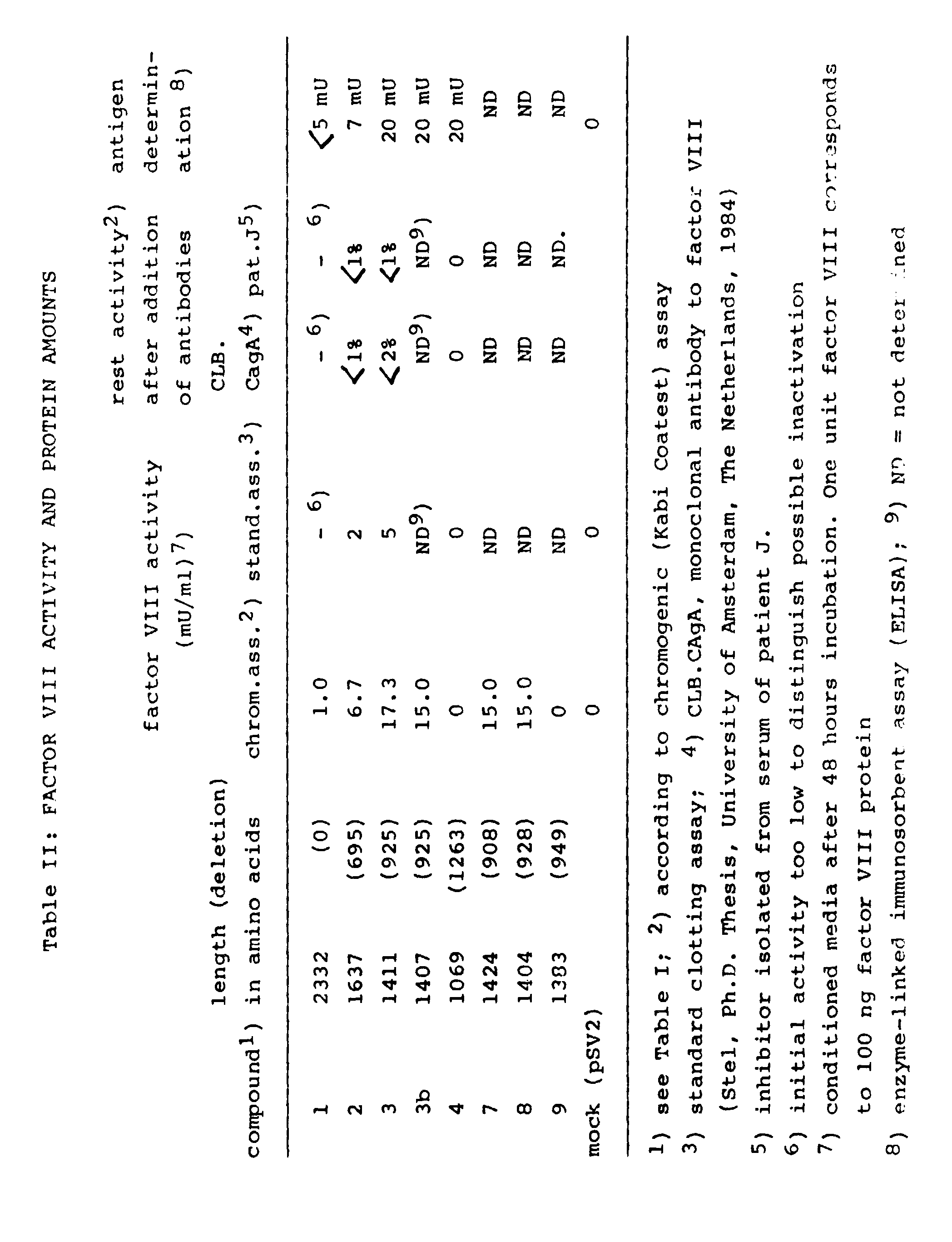

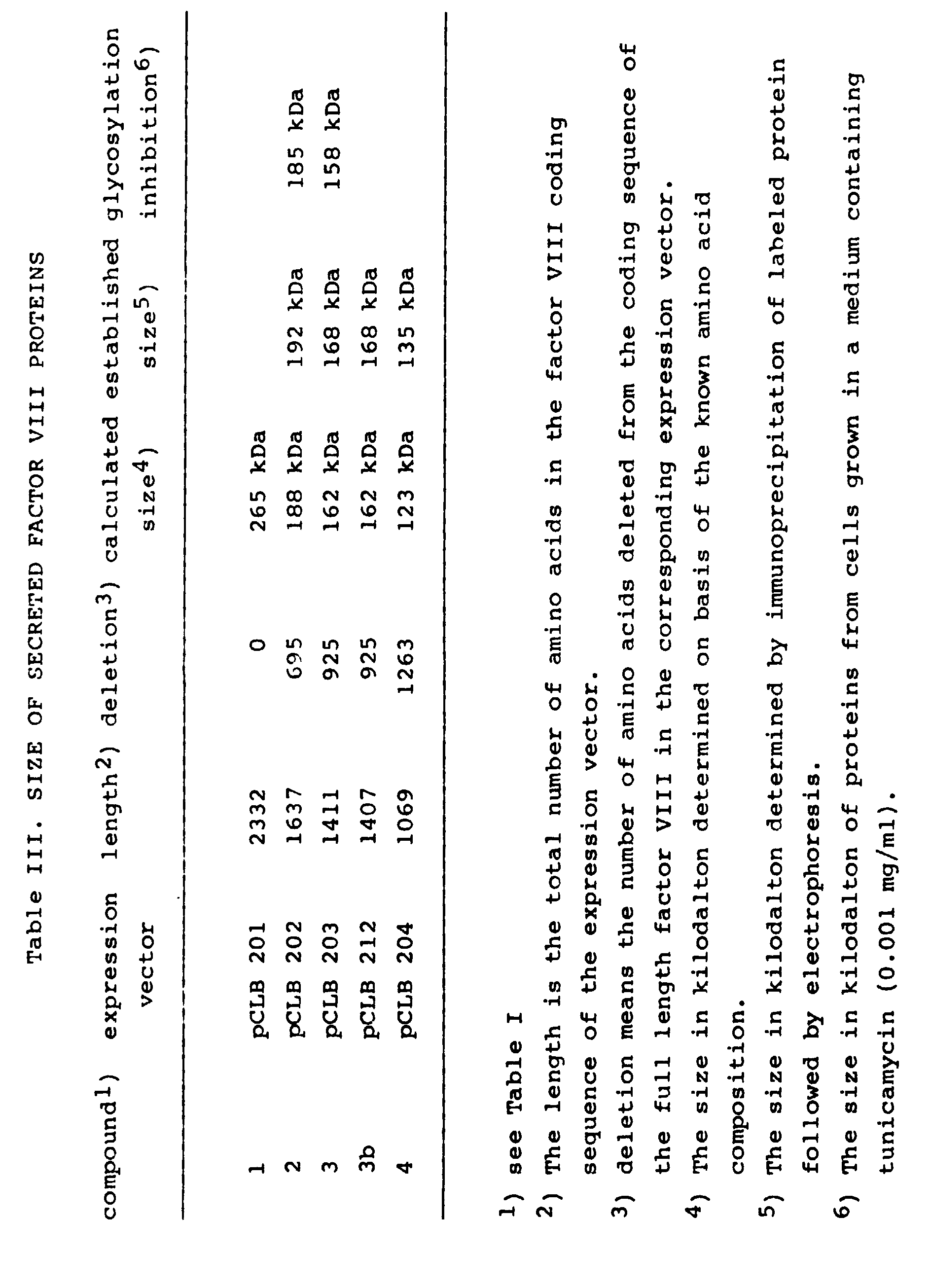

## Claims (Claims for the following Contracting State(s): AT, BE, CH, LI, DE, FR, GB, IT, LU, NL, SE)

1. A protein having biological activity of Factor VIII and having an amino acid sequence corresponding to the amino acid sequence of Factor VIII except for a deletion of the amino acid sequence Ser-741 through Ile-1668.

2. A protein according to claim 1 having a deletion of at least part of the amino acid sequence Lys-713 through Arg-740 and an insertion of the amino acid sequence Pro-Arg-Val-Ala, as a linker sequence in the site of said deletion.

3. A recombinat DNA molecule comprising a DNA sequence coding for a protein as defined in claim 1 or 2.

4. An expression vector comprising, in the direction of transcription, a transcriptional regulatory region and a translational initiation region functional in a host cell, a DNA sequence coding for a protein as defined in claim 1 or 2 and translational and transcriptional termination regions functional in said host cell, wherein expression of said DNA sequence is regulated by said initiation and termination regions.

5. A transformed cell and progeny thereof, said cell comprising an expression vector comprising, in the direction of transcription, a transcriptional regulatory region and a translational initiation region functional in a host cell, a DNA sequence coding for a protein as defined in claim 1 or 2 and translational and transcriptional termination regions functional in said host cell, wherein expression of said DNA sequence is regulated by said initiation and termination regions.

6. A transformed cell and progeny thereof according to claim 5, wherein said cell is a mammalian cell.

7. A transformed cell and progeny thereof according to claim 6, wherein said cell is a COS cell, a CHO cell or a C127 cell.

8. A transformed cell and progeny thereof according to claim 5, wherein said cell is a prokaryotic cell or a yeast cell.

9. A transformed cell and progeny thereof according to claim 8, wherein said cell is of a Bacillus or Kluyveromyces species.

10. A method for preparing a protein which is a deletion mutant of Factor VIII having biological activity of Factor VIII, said method comprising growing, in a nutrient medium, a host cell comprising an expression vector comprising, in the direction of transcription, a transcriptional regulatory region and a translational initiation region functional in a host cell, a DNA sequence coding for a protein as defined in claim 1 or 2 and translational and transcriptional termination regions functional in said host cell, wherein expression of said DNA sequence is regulated by said initiation and termination regions, and isolating said protein.

11. A Factor VIII deficiency symptom-alleviating composition comprising a symptom-alleviating amount of a protein which is a deletion mutant of Factor VIII having biological activity of Factor VIII as defined in claim 1 or 2 and a physiologically acceptable carrier.

## Claims (Claims for the following Contracting State(s): GR, ES)

1. A protein having biological activity of Factor VIII and having an amino acid sequence corresponding to the amino acid sequence of Factor VIII except for a deletion of the amino acid sequence Ser-741 through Ile-1668.

2. A protein according to claim 1 having a deletion of at least part of the amino acid sequence Lys-713 through Arg-740 and an insertion of the amino acid sequence Pro-Arg-Val-Ala, as a linker sequence in the site of said deletion.

3. A recombinant DNA molecule comprising a DNA sequence coding for a protein as defined in claim 1 or 2.

4. An expression vector comprising, in the direction of transcription, a transcriptional regulatory region and a translational initiation region functional in a host cell, a DNA sequence coding for a protein as defined in claim 1 or 2 and translational and transcriptional termination regions functional in said host cell, wherein expression of said DNA sequence is regulated by said initiation and termination regions.

5. A transformed cell and progeny thereof, said cell comprising an expression vector comprising, in the direction of transcription, a transcriptional regulatory region and a translational intitiation region functional in a host cell, a DNA sequence coding for a protein as defined in claim 1 or 2 and translational and transcriptional termination regions functional in said host cell, wherein expression of said DNA sequence is regulated by said initiation and termination regions.

6. A transformed cell and progeny thereof according to claim 5, wherein said cell is a mammalian cell.

7. A transformed cell and progeny thereof according to claim 6, wherein said cell is a COS cell, a CHO cell or a C127 cell.

8. A transformed cell and progeny thereof according to claim 5, wherein said cell is a prokaryotic cell or a yeast cell.

9. A transformed cell and progeny thereof according to claim 8, wherein said cell is of a Bacillus or Kluyveromyces species.

10. A method for preparing a protein which is a deletion mutant of Factor VIII having biological activity of Factor VIII, said method comprising growing, in a nutrient medium, a host cell comprising an expression vector comprising, in the direction of transcription, a transcriptional regulatory region and a translational initiation region functional in a host cell, a DNA sequence coding for a protein as defined in claim 1 or 2 and translational and transcriptional termination regions functional in said host cell, wherein expression of said DNA sequence is regulated by said initiation and termination regions, and isolating said protein.

11. A Factor VIII deficiency symptom-alleviating composition comprising a symptom-alleviating amount of a protein which is a deletion mutant of Factor VIII having biological activity of Factor VIII as defined in claim 1 or 2 and a physiologically acceptable carrier.

12. A method for preparing a protein which is a deletion mutant of Factor VIII having biological activity of Factor VIII, said method comprising growing, in a nutrient medium, a host cell comprising an expression vector comprising, in the direction of transcription, a transcriptional regulatory region and a translational initiation region functional in the host cell, a DNA sequence coding for said protein corresponding to the amino acid sequence of Factor VIII except for a deletion of the amino acid sequence Ser-741 through Ile-1668 and/or deletion of at least part of the amino acid sequence Lys-713 through Arg-740 and an insertion of the amino acid sequence Pro-Arg-Val-Ala, as a linker sequence in the site of said deletion, and transcriptional termination regions functional in said host cell, wherein expression of said DNA sequence is regulated by said initiation and termination regions, and isolating said protein.

13. A method according to claim 1, wherein said cell is a mammalian cell.

14. A method according to claim 2, wherein said cell is a COS cell, a CEO cell or a C127 cell.

## Patentansprüche (Patentansprüche für folgende(n) Vertragsstaat(en): AT, BE, CH, LI, DE, FR, GB, IT, LU, NL, SE)

1. Protein mit biologischer Faktor VIII-Aktivität und mit einer Aminosäuresequenz, entsprechend der Aminosäuresequenz von Faktor VIII mit Ausnahme einer Deletion der Aminosäuresequenz Ser-741 bis Ile-1668.

2. Protein nach Anspruch 1 mit einer Deletion von mindestens einem Teil der Aminosäuresequenz Lys-713 bis Arg-740 und einer Insertion der Aminosäuresequenz Pro-Arg-Val-Ala als Linkersequenz an der Deletionsstelle.

3. Rekombinantes DNA-Molekül, umfassend eine DNA-Sequenz, welche für ein Protein gemäß Anspruch 1 oder 2 kodiert.

4. Expressionsvektor, umfassend, in Transkriptionsrichtung eine transkriptionale regulatorische Region und eine translationale Initiationsregion, die in einer Wirtszelle funktional sind, eine DNA-Sequenz, die für ein Protein gemäß Anspruch 1 oder 2 kodiert, und translationale und transkriptionale Terminationsregionen, die in Wirtszelle funktional sind, worin die Expression der DNA-Sequenz durch die Initiations- und Terminationsregionen reguliert wird.

5. Transformierte Zelle und Progen davon, wobei die Zelle einen Expressionsvektor enthält, der, in Transkriptionsrichtung, eine transkriptionale regulatorische Region und eine translationale Initiationsregion, die in einer Wirtszelle funktional sind, eine DNA-Sequenz, die für ein Protein gemäß Anspruch 1 oder 2 und translationale und transkriptionale Tenninationsregionen, die in der Wirtszelle funktional sind, umfaßt, worin die Expression der DNA-Sequenz durch die Initiations- und Terminationsregionen reguliert wird.

6. Tranformierte Zelle und Progen davon nach Anspruch 5, worin die Zelle eine Säugerzelle ist.

7. Transformierte Zelle und Progen davon nach Anspruch 6, worin die Zelle eine COS-Zelle, eine CEO-Zelle oder eine C127-Zelle ist.

8. Transformierte Zelle und Progen davon gemäß Anspruch 5, worin die Zelle eine prokaryontische oder eine Hefezelle ist.

9. Transformierte Zelle und Progen davon gemäß Anspruch 1, worin die Zelle zur Spezies **Bacillus** oder **Kluyveromyces** gehört.

10. Verfahren zur Herstellung eines Proteins, welches eine Deletionsmutante des Faktors VIII mit biologischen Aktivität des Faktors VIII ist, wobei das Verfahren das Wachsenlassen in einem Nährmedium von einer Wirtszelle, welche einen Expressionsvektor enthält, der in Transkriptionsrichtung, eine transkriptionale regulatorische Region und eine translationale Initiationsregion, die in der Wirtszelle funktional sind, eine DNA-Sequenz, kodierend für ein Protein nach Anspruch 1 oder 2, und translationale und transkriptionale Terminationsregionen, die in der Wirtszelle funktional sind, umfaßt, worin die Expression der DNA-Sequenz durch die Initiations- und Terminationsregionen reguliert wird, und Isolieren des Proteins umfaßt.

11. Faktor VIII -Mangelsymptom-lindernde Zusammensetzung, umfassend eine symptomlindernde Menge des Proteins, welches eine Deletionsmutante des Faktors VIII mit der biologischen Aktivität des Faktors VIII gemäß Anspruch 1 oder 2 ist, und einen physiologisch verträglichen Träger.

## Patentansprüche (Patentansprüche für folgende(n) Vertragsstaat(en): GR, ES)

1. Protein mit biologischer Faktor VIII-Aktivität und mit einer Aminosäuresequenz, entsprechend der Aminosäuresequenz von Faktor VIII mit Ausnahme einer Deletion der Aminosäuresequenz Ser-741 bis Ile-1668.

2. Protein nach Anspruch 1 mit einer Deletion von mindestens einem Teil der Aminosäuresequenz Lys-713 bis Arg-740 und einer Insertion der Aminosäuresequenz Pro-Arg-Val-Ala als Linkersequenz an der Deletionsstelle.

3. Rekombinantes DNA-Molekül, umfassend eine DNA-Sequenz, welche für ein Protein gemäß Anspruch 1 oder 2 kodiert.

4. Expressionsvektor, umfassend, in Transkriptionsrichtung eine transkriptionale regulatorische Region und eine translationale Initiationsregion, die in einer Wirtszelle funktional sind, eine DNA-Sequenz, die für ein Protein gemäß Anspruch 1 oder 2 kodiert, und translationale und transkriptionale Terminationsregionen, die in wirtszelle funktional sind, worin die Expression der DNA-Sequenz durch die Initiations- und Terminationsregionen reguliert wird.

5. Transformierte Zelle und Progen davon, wobei die Zelle einen Expressionsvektor enthält, der, in Transkriptionsrichtung, eine transkriptionale regulatorische Region und eine translationale Initiationsregion, die in einer Wirtszelle funktional sind, eine DNA-Sequenz, die für ein Protein gemäß Anspruch 1 oder 2 und translationale und transkriptionale Terminationsregionen, die in der Wirtszelle funktional sind, umfaßt, worin die Expression der DNA-Sequenz durch die Initiations- und Terminationsregionen reguliert wird.

6. Tranformierte Zelle und Progen davon nach Anspruch 5, worin die Zelle eine Säugerzelle ist.

7. Transformierte Zelle und Progen davon nach Anspruch 6, worin die Zelle eine COS-Zelle, eine CEO-Zelle oder eine C127-Zelle ist.

8. Transformierte Zelle und Progen davon gemäß Anspruch 5, worin die Zelle eine prokaryontische oder eine Hefezelle ist.

9. Transformierte Zelle und Progen davon gemäß Anspruch 1, worin die Zelle zur Spezies **Bacillus** oder **Kluyveromyces** gehört.

10. Verfahren zur Herstellung eines Proteins, welches eine Deletionsmutante des Faktors VIII mit biologischen Aktivität des Faktors VIII ist, wobei das Verfahren das Wachsenlassen in einem Nährmedium von einer Wirtszelle, welche einen Expressionsvektor enthält, der in Transkriptionsrichtung, eine transkriptionale regulatorische Region und eine translationale Initiationsregion, die in der Wirtszelle funktional sind, eine DNA-Sequenz, kodierend für ein Protein nach Anspruch 1 oder 2, und translationale und transkriptionale Terminationsregionen, die in der Wirtszelle funktional sind, umfaßt, worin die Expression der DNA-Sequenz durch die Initiations- und Terminationsregionen reguliert wird, und Isolieren des Proteins umfaßt.

11. Faktor VIII -Mangelsymptom-lindernde Zusammensetzung, umfassend eine symptomlindernde Menge des Proteins, welches eine Deletionsmutante des Faktors VIII mit der biologischen Aktivität des Faktors VIII gemäß Anspruch 1 oder 2 ist, und einen physiologisch verträglichen Träger.

12. Verfahren zur Herstellung eines Proteins, welches eine Deletionsmutante von Faktor VIII mit der biologischen Aktivität des Faktors VIII ist, wobei das Verfahren das Wachsenlassen in einem Nährmedium von einer Wirtszelle, die einen Expressionsvektor enthält, der, in Transkriptionsrichtung eine transkriptionale regulatorische Region und eine translationale Initiationsregion, die in der Wirtszelle funktional sind, eine DNA-Sequenz, welche für das Protein entsprechend der Aminosäuresequenz des Faktors VIII mit Ausnahme einer Deletion der Aminosäuresequenz Ser-741 bis Ile-1668 und/oder einer Deletion von mindestens einem Teil der Aminosäuresequenz Lys-713 bis Arg-740 und einer Insertion der Aminosäuresequenz Pro-Arg-Val-Ala als Linkersequenz an der Stelle der Deletion, und transkriptionale Terminationsregionen, die in der Wirtszelle funktional sind, umfaßt, worin die Expression der DNA-Sequenz durch die Initiations- und Terminationsregionen reguliert wird, und Isolieren des Proteins umfaßt.

13. Verfahren nach Anspruch 1, worin die Zelle eine Säugerzelle ist.

14. Verfahren nach Anspruch 2, worin die Zelle eine COS-Zelle, eine CEO-Zelle oder eine C127-Zelle ist.

## Revendications (Revendications pour l'(les) Etat(s) contractant(s) suivant(s): AT, BE, CH, LI, DE, FR, GB, IT, LU, NL, SE)

1. Protéine à activité biologique du facteur VIII et ayant une séquence d'aminoacides correspondant à la séquence d'aminoacides du facteur VIII à l'exception d'une délétion de la séquence d'aminoacides Ser-741 à Ile-1668.

2. Protéine suivant la revendication 1, présentant une délétion d'au moins une partie de la séquence d'aminoacides Lys-713 à Arg-740 et une insertion de la séquence d'aminoacides Pro-Arg-Val-Ala, comme séquence de liaison dans le site de ladite délétion.

3. Molécule d'ADN recombinant comprenant une séquence d'ADN codant pour une protéine répondant à la définition suivant la revendication 1 ou 2.

4. Vecteur d'expression comprenant, dans le sens de la transcription, une région régulatrice de transcription et une région d'initiation de traduction fonctionnelles dans une cellule hôte, une séquence d'ADN codant pour une protéine répondant à la définition suivant la revendication 1 ou 2 et des régions de terminaison de traduction et de transcription fonctionnelles dans ladite cellule hôte, l'expression de ladite séquence d'ADN étant régulée par lesdites régions d'initiation et de terminaison.

5. Cellule transformée et sa descendance, ladite cellule comprenant un vecteur d'expression renfermant, dans le sens de la transcription, une région régulatrice de transcription et une région d'initiation de traduction fonctionnelles dans une cellule hôte, une séquence d'ADN codant pour une protéine répondant à la définition suivant la revendication 1 ou 2 et des régions de terminaison de traduction et de transcription fonctionnelles dans ladite cellule hôte, l'expression de ladite séquence d'ADN étant régulée par lesdites régions d'initiation et de terminaison.

6. Cellule transformée et sa descendance suivant la revendication 5, dans lesquelles ladite cellule est une cellule de mammifère.

7. Cellule transformée et sa descendance suivant la revendication 6, dans lesquelles ladite cellule est une cellule COS, une cellule CHO ou une cellule C127.

8. Cellule transformée et sa descendance suivant la revendication 5, dans lesquelles ladite cellule est une cellule procariotique ou une cellule de levure.

9. Cellule transformée et sa descendance suivant la revendication 8, dans lesquelles ladite cellule est une espèce du genre Bacillus ou Kluyveromyces.

10. Procédé de préparation d'une protéine qui est un mutant par délétion du facteur VIII à activité biologique du facteur VIII, ledit procédé comprenant la culture, dans un milieu nutritif, d'une cellule hôte comprenant un vecteur d'expression renfermant, dans le sens de la transcription, une région régulatrice de transcription et une région d'initiation de traduction fonctionnelles dans une cellule hôte, une séquence d'ADN codant pour une protéine répondant à la définition suivant la revendication 1 ou 2 et des régions de terminaison de traduction et de transcription fonctionnelles dans ladite cellule hôte, l'expression de ladite séquence d'ADN étant régulée par lesdites régions d'initiation et de terminaison, et l'isolement de ladite protéine.

11. Composition réduisant les symptômes de déficience en facteur VIII, comprenant une quantité, ayant pour effet de réduire les symptômes, d'une protéine qui est un mutant par délétion du facteur VIII à activité biologique du facteur VIII répondant à la définition suivant la revendication 1 ou 2 et un support physiologiquement acceptable.

## Revendications (Revendications pour l'(les) Etat(s) contractant(s) suivant(s): GR, ES)

1. Protéine à activité biologique du facteur VIII et ayant une séquence d'aminoacides correspondant à la séquence d'aminoacides du facteur VIII à l'exception d'une délétion de la séquence d'aminoacides Ser-741 à Ile-1668.

2. Protéine suivant la revendication 1, présentant une délétion d'au moins une partie de la séquence d'aminoacides Lys-713 à Arg-740 et une insertion de la séquence d'aminoacides Pro-Arg-Val-Ala, comme séquence de liaison dans le site de ladite délétion.

3. Molécule d'ADN recombinant comprenant une séquence d'ADN codant pour une protéine répondant à la définition suivant la revendication 1 ou 2.

4. Vecteur d'expression comprenant, dans le sens de la transcription, une région de régulation de transcription et une région d'initiation de traduction fonctionnelles dans une cellule hôte, une séquence d'ADN codant pour une protéine répondant à la définition suivant la revendication 1 ou 2 et des régions de terminaison de traduction et de transcription fonctionnelles dans ladite cellule hôte, l'expression de ladite séquence d'ADN étant régulée par lesdites régions d'initiation et de terminaison.

5. Cellule transformée et sa descendance, ladite cellule comprenant un vecteur d'expression renfermant, dans le sens de la transcription, une région régulatrice de transcription et une région d'initiation de traduction fonctionnelles dans une cellule hôte, une séquence d'ADN codant pour une protéine répondant à la définition suivant la revendication 1 ou 2 et des régions de terminaison de traduction et de transcription fonctionnelles dans ladite cellule hôte, l'expression de ladite séquence d'ADN étant régulée par lesdites régions d'initiation et de terminaison.

6. Cellule transformée et sa descendance suivant la revendication 5, dans lesquelles ladite cellule est une cellule de mammifère.

7. Cellule transformée et sa descendance suivant la revendication 6, dans lesquelles ladite cellule est une cellule COS, une cellule CHO ou une cellule C127.

8. Cellule transformée et sa descendance suivant la revendication 5, dans lesquelles ladite cellule est une cellule procariotique ou une cellule de levure.

9. Cellule transformée et sa descendance suivant la revendication 8, dans lesquelles ladite cellule est une espèce du genre Bacillus ou Kluyveromyces.

10. Procédé de préparation d'une protéine qui est un mutant par délétion du facteur VIII ayant l'activité biologique du facteur VIII, ledit procédé comprenant la culture, dans un milieu nutritif, d'une cellule hôte comprenant un vecteur d'expression renfermant, dans le sens de la transcription, une région régulatrice de transcription et une région d'initiation de traduction fonctionnelles dans une cellule hôte, une séquence d'ADN codant pour une protéine répondant à la définition suivant la revendication 1 ou 2 et des régions de terminaison de traduction et de transcription fonctionnelles dans ladite cellule hôte, l'expression de ladite séquence d'ADN étant régulée par lesdites régions d'initiation et de terminaison, et l'isolement de ladite protéine.

11. Composition réduisant les symptômes de déficience en facteur VIII, comprenant une quantité, ayant pour effet de réduire les symptômes, d'une protéine qui est un mutant par délétion du facteur VIII ayant l'activité biologique du facteur VIII répondant à la définition suivant la revendication 1 ou 2 et un support physiologiquement acceptable.

12. Procédé de préparation d'une protéine qui est un mutant par délétion du facteur VIII ayant l'activité biologique du facteur VIII, ledit procédé comprenant la culture, dans un milieu nutritif, d'une cellule hôte comprenant un vecteur d'expression renfermant, dans le sens de la transcription, une région régulatrice de transcription et une région d'initiation de traduction fonctionnelles dans la cellule hôte, une séquence d'ADN codant pour ladite protéine correspondant à la séquence d'aminoacides du facteur VIII à l'exception d'une délétion de la séquence d'aminoacides Ser-741 à Ile-1668 et/ou d'une délétion d'au moins une partie de la séquence d'aminoacides Lys-713 à Arg-740 et d'une insertion de la séquence d'aminoacides Pro-Arg-Val-Ala, comme séquence de liaison dans le site de ladite délétion, et des régions de terminaison de transcription fonctionnelles dans ladite cellule hôte, l'expression de ladite séquence d'ADN étant régulée par lesdites régions d'initiation et de terminaison, et l'isolement de ladite protéine.

13. Procédé suivant la revendication 1, dans lequel la cellule est une cellule de mammifère.

14. Procédé suivant la revendication 2, dans lequel la cellule est une cellule COS, une cellule CHO ou une cellule C127.
